# EUROPEAN PATENT SPECIFICATION

(11) **EP 3 308 164 B1**
(45) Date of publication and mention of the grant of the patent: **21.08.2019**
(21) Application number: 16732512.5
(22) Date of filing: 10.06.2016
(51) Int. Cl.: G01N 33/50, C12Q 1/68

(54) **HIGH THROUGHPUT OPTIMIZATION OF CONTENT-LOADED NANOPARTICLES**
HOCHDURCHSATZOPTIMIERUNG INHALTSGELADENER NANOPARTIKEL
OPTIMISATION À HAUT RENDEMENT DE NANOPARTICULES CHARGÉES EN CONTENU

(30) Priority: 10.06.2015 EP 15171429
(43) Date of publication of application: 18.04.2018
(73) Proprietor: Danmarks Tekniske Universitet, 2800 Kgs. Lyngby (DK)
(72) Inventor: HADRUP, Sine, Reker, 2830 Virum (DK); LYNGSØ, Christina, 2830 Virum (DK); JAKOBSEN, Søren, Nyboe, 2920 Charlottenlund (DK)
(74) Representative: Plougmann Vingtoft a/s
(86) International application number: PCT/EP2016/063314
(87) International publication number: WO 2016/198609

(56) References cited:
- WO-A1-2012/016357
- US-A1- 2014 057 276
- LOO JACKY F C ET AL: "An aptamer-based bio-barcode assay with isothermal recombinase polymerase amplification for cytochrome-cdetection and anti-cancer drug screening", TALANTA, vol. 115, 28 April 2013 (2013-04-28), pages 159-165, XP028720522, ISSN: 0039-9140, DOI: 10.1016/J.TALANTA.2013.04.051
- M. J. FELDHAUS ET AL: "Oligonucleotide-conjugated beads for transdominant genetic experiments", NUCLEIC ACIDS RESEARCH, vol. 28, no. 2, 15 January 2000 (2000-01-15), pages 534-543, XP055213661, DOI: 10.1093/nar/28.2.534
- RULE GEOFFREY S ET AL: "Rapid method for visual identification of specific DNA sequences based on DNA-tagged liposomes", CLINICAL CHEMISTRY, AMERICAN ASSOCIATION FOR CLINICAL CHEMISTRY, WASHINGTON, DC, vol. 42, no. 8 PART 1, 1 January 1996 (1996-01-01), pages 1206-1209, XP002559997, ISSN: 0009-9147
- DONGBUM KIM ET AL: "Production of antibodies with peptide-CpG-DNA-liposome complex without carriers", BMC IMMUNOLOGY, BIOMED CENTRAL, LONDON, GB, vol. 12, no. 1, 18 May 2011 (2011-05-18), page 29, XP021099649, ISSN: 1471-2172, DOI: 10.1186/1471-2172-12-29

## Description

### Technical field of the invention

The present invention relates to tagged particles and the identification and characterization of particles based on their tag. In particular, the present invention relates to a method for the production of a multitude of uniquely tagged particles comprised of a range of components selected from the group consisting of carriers, cargo and surface molecules, and the identification of such particles causing a specific effect/change in a sample, such as certain tissues/cell types.

### Background of the invention

Drug development is very costly and many drugs fail clinically or commercially due to suboptimal efficacy and/or unacceptable adverse effects. Consequently, much effort is put into research and development of new and more effective drug formulations that are optimized to maximize effect, minimize side effects and preferentially deliver drugs at their intended site of action in the body.

Recently, much focus has been given to particulate drug formulations which may be targeted to specific cell types or anatomical sites to improve the therapeutic window. However, efficient optimization of particulate formulations is currenty not possible due to the sheer number of different potential formulation components, such as polymers, drug variants including pro-drugs, surface molecules etc. There is therefore a need for efficient methods for synthesizing, such as combinatorially synthesizing, large libraries of particle formulations followed by screening to find the best variants and deconvolution of minute amounts of such variants to identify their specific components.

It has previously been proposed that screening of large libraries of compounds such as small molecules, peptides or antibodies and subsequent decoding of the barcode to identify individual, partitioned compounds can be accomplished by tagging every compound in the library with a unique tag or barcode, e.g. made of DNA. Most of these approaches are however limited to affinity screening and cannot address issues of formulation stability, pharmacokinetics and pharmacodynamics, effect/change on targets in their natural environment, effect/change on cells, including primary cells, effect/change on tissue of an organism, targeting to specific cells and anatomical sites etc.

Oligonucleotides have previously been used in combination with liposomes, but primarily as a means of obtaining a desirable interaction, e.g. fusion of liposomes or sorting of liposomes, and not as a tool for screening of nanoparticles.

Hence, a method for identifying and optimizing the influence of the individual components of pharmaceutical nanoparticles on cells or in vivo would be of great value. In particular, a more efficient and reliable method for producing tagged multicomponent nanoparticles combined with a complementary screening assay that operates in a high throughput manner would be advantageous.

### Summary of the invention

A first aspect of the present invention is a tagged particle comprising at least one component from each of a)-c):
a) a carrier (P) selected from the group consisting of liposomes, polymeric particles, micelles, albumin complexes, dendrimers, metal colloids and ceramics,
b) a cargo molecule (K) selected from the group consisting of drugs, oligonucleotides, proteins, antibodies, radioisotopes, markers, metal ions, adjuvants, organic molecules, small molecules and cytokines, and
c) a surface molecule molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
and an oligonucleotide tag comprising one oligonucleotide tag sub-segment (p, k, t, z) for each component from a)-c).

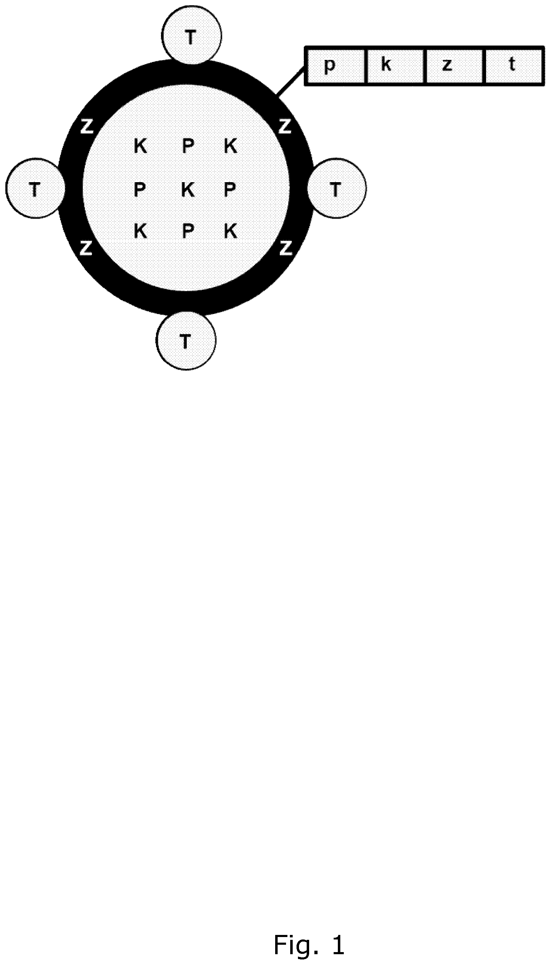

Figure 1 exemplified this aspect.

A second aspect of the present invention is a split and mix method for the production of a plurality of tagged particles, the method comprising at least the step of:
i. mixing one component from either of a)-c):
   a) a carrier (P) selected from the group consisting of liposomes, polymeric particles, micelles, albumin complexes, dendrimers, metal colloids and ceramics,
   b) a cargo molecule (K) selected from the group consisting of drugs, oligonucleotides, proteins, antibodies, radioisotopes, markers, metal ions, adjuvants, organic molecules, small molecules and cytokines, and
   c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
   with an oligonucleotide tag sub-segment (p, k, t, z) to form a first solution of first generation tagged particles,
ii. splitting said first solution of first generation tagged particles into two or more first solutions of first generation tagged particles,
iii. mixing said two or more first solutions of first generation tagged particles with one component from either of a)-c) and an oligonucleotide tag sub-segment (p, k, t, z) to form two or more second solutions of second generation tagged particles,
iv. splitting said second solution of second generation tagged particles into two or more second solutions of second generation tagged particles,
v. mixing said two or more second solutions of second generation tagged particles with one component from either of a)-c) and an oligonucleotide tag sub-segment (p, k, t, z) to form two or more third solutions of third generation tagged particles,
and wherein the plurality of tagged particles resulting from step v) comprise at least one component from each of a)-c).

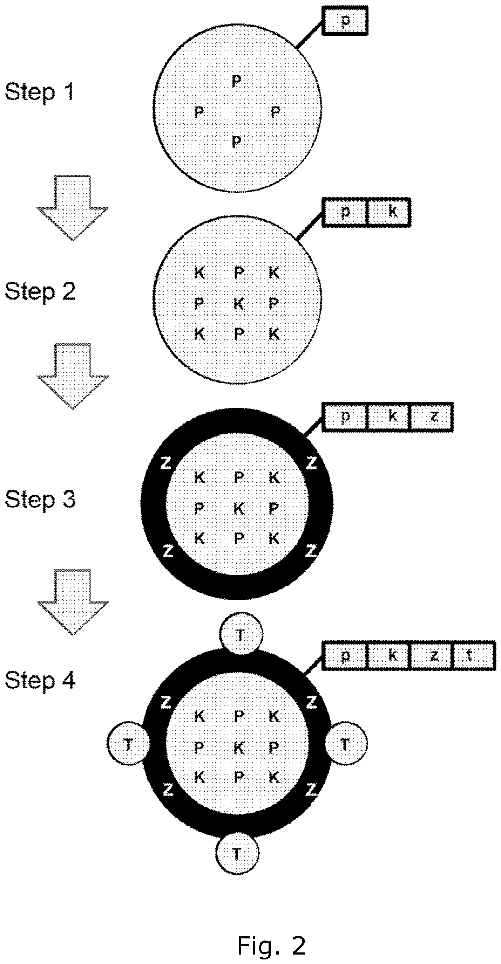

Figure 2 exemplifies this aspect.

A third aspect of the present invention is a method for the identification of individual components of a particle, the method comprising the steps of:
i. providing a plurality of tagged particles comprising at least one component from each of a)-c):
   a) a carrier (P) selected from the group consisting of liposomes, polymeric particles, micelles, albumin complexes, dendrimers, metal colloids and ceramics,
   b) a cargo molecule (K) selected from the group consisting of drugs, oligonucleotides, proteins, antibodies, radioisotopes, markers, metal ions, adjuvants, organic molecules, small molecules and cytokines, and
   c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
   and an oligonucleotide tag comprising one oligonucleotide tag sub-segment (p, k, t, z) for each component from a)-c),
ii. contacting said plurality of tagged particles with a sample,
iii. evaluating the ability of the tagged particles to induce biological, morphological, chemical, biochemical, catalytic, physical and/or physiological changes on the sample,
iv. identifying one or more tagged particles causing a specific change,
v. recovering from the sample said one or more tagged particles causing the specific change, and
vi. identifying by their oligonucleotide tag the at least one component from each of a)-c) of said one or more recovered tagged particles.

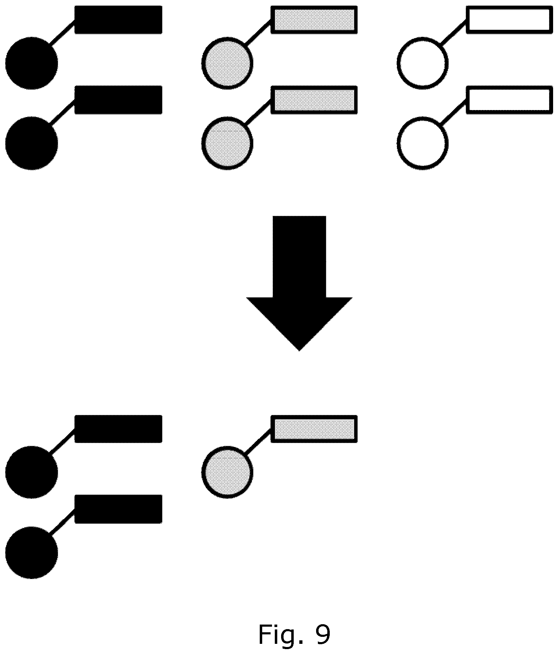

Figure 9 exemplifies this aspect.

### Brief description of the figures

Figure 1: Composition of tagged particle with carrier (P), cargo (K), surface molecules (Z, T) and tag subsets (p, k, z, t) which encodes corresponding components written in uppercase.
Figure 2: Formation of tagged particle
   Step 1; Formation of particle from carrier components (P). Addition of tag (p) encoding carrier.
   Step 2; Addition of cargo (K). Addition of tag (k) encoding cargo.
   Step 3; Addition of surface layer (Z). Addition of tag (z) encoding surface layer.
   Step 4; Addition of targeting surface molecules (T). Addition of tags (t) encoding targeting surface molecules.

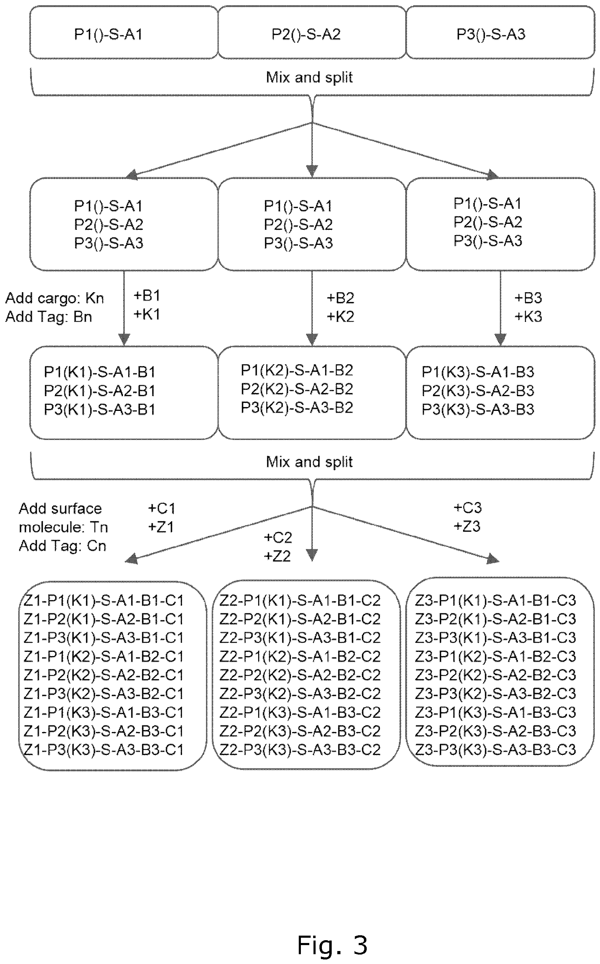

Figure 3: Mix and split synthesis of tagged particles.
   In a first step, three different particles (P1, P2, P3) are formed, each with a tag (S) anchored in the particle and a tag (A1, A2, A3) encoding the carrier of each particle.
   Particles are mixed and split into three new compartments. In each compartment a cargo (K1, K2, K3) is added to particles and tags (B1, B2, B3) encoding cargo are added. Then, the content of all compartments is mixed and split into three new compartments.
   In each compartment, surface molecules (Z1, Z2, Z3) are added to the particles and tags (C1, C2, C3) are added to encoded the surface molecules.
   Finally (not shown), the content of all compartments is mixed and a terminal tag (T) is added. The mixture is then ready for screening/assays.
   Legend: Pn() denotes carrier n, empty paranthesis denotes no cargo loaded. Entities in parentheses denotes cargo associated with carrier. A dash denotes a link. Zn denotes cargo n. S denotes starting oligo. An denotes tag n encoding carrier Pn. Bn denotes tag n encoding cargo Zn. Cn denotes tag n encoding surface molecule Tn. Annealing sequences are omitted for clarity.

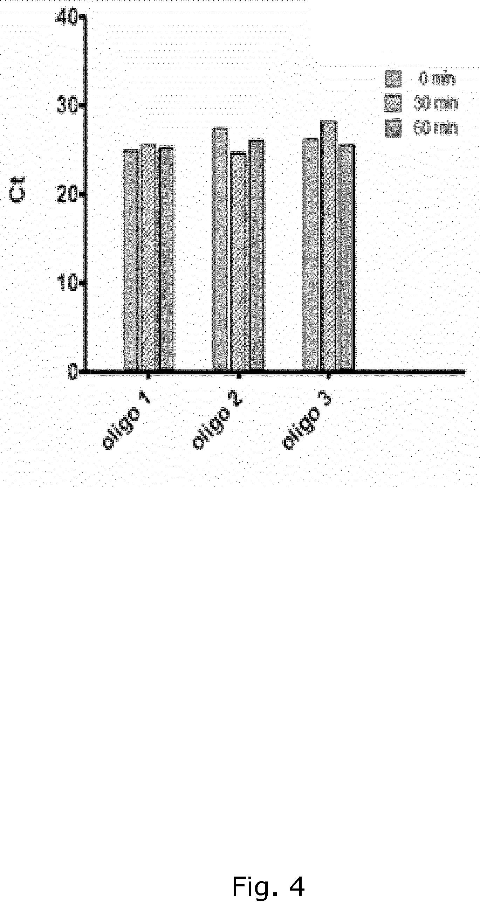

Figure 4: Stability of particle tags in a sample.
   The figure shows the stability of DNA tags in peripheral blood. DNA tags were recovered and amplified by qPCR after different incubation periods (0, 30 and 60 min) with peripheral blood lymphocytes. Measurement were repeated with three different DNA oligo designs.

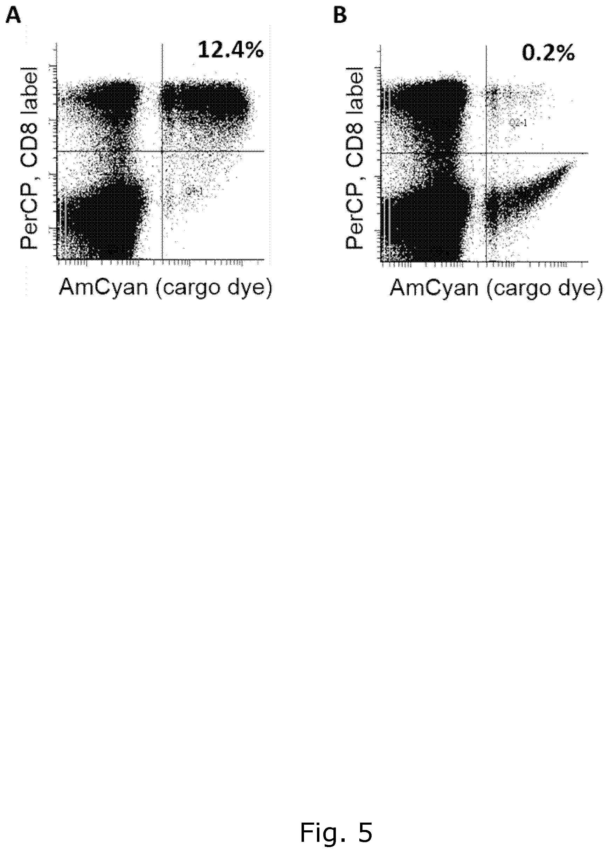

Figure 5: Targeted delivery of particles to specific cell type.
   Specific delivery of polystyrene beads coated with anti-CD8 antibodies to CD8 positive lymphocytes. The figure shows a comparison between coated and non-coated polystyrene beads. Data is collected by flow cytometry in which anti-CD8 PerCp are added for visualization.

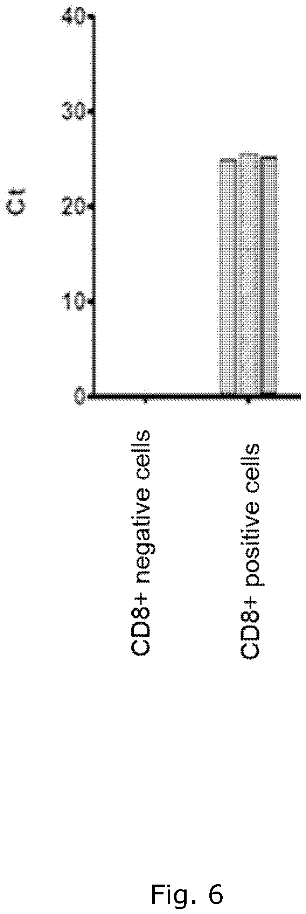

Figure 6: Identification of particle composition through deconvolution of tag.

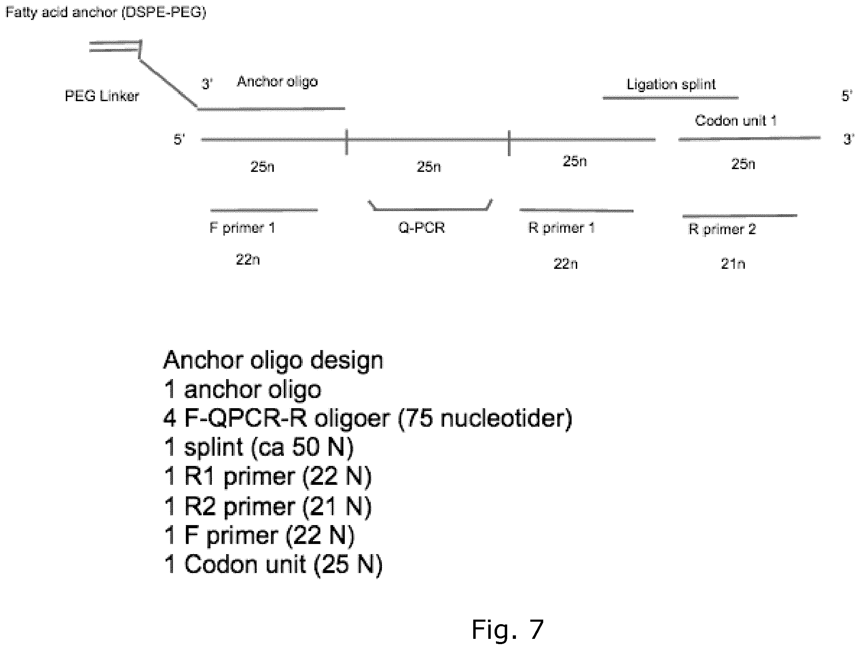

Figure 7: Tag design

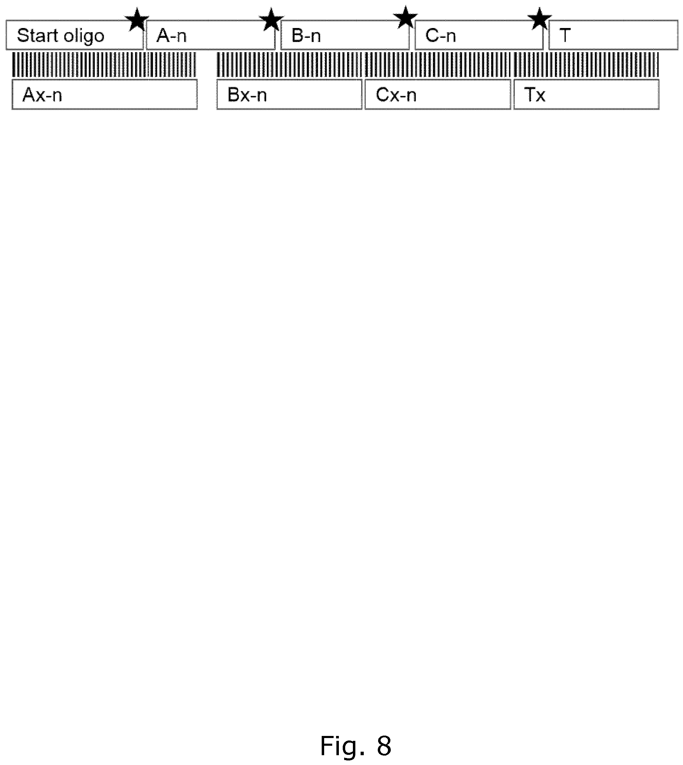

Figure 8: Mix and split tag design.
   Top strand is shown 3'-5'. Botton strand is shown 5'-3'. Stars correspond to 5' phosphorylated nucleotides. Vertical lines (straight hashes) indicate annealing between top and bottom strand tag oligos.
Figure 9: Screening of a library of tagged particles

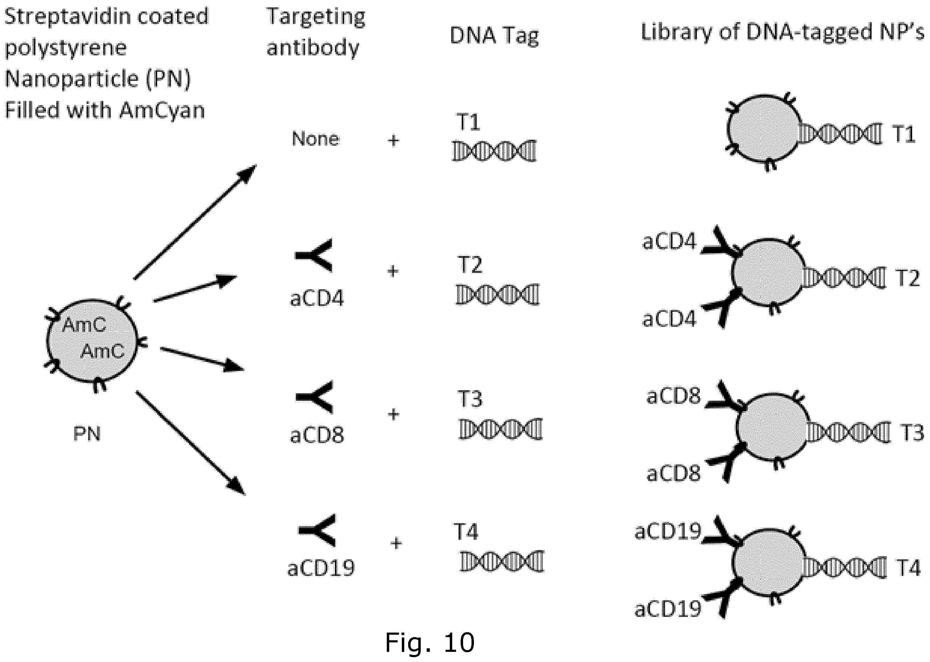

Figure 10: Synthesis of a 4-member DNA-tagged library of polystyrene nanoparticles (PN) with AmCyan (AmC) as cargo and targeting antibodies as surface molecules. First, particles comprising streptavidin on their surface are coated with biotinylated (B) anti-CD4 antibody, anti-CD8 antibody, anti-CD19 antibody or no antibody respectively. Then, particles are linked to DNA-tags encoding the surface molecules. The result is a library of DNA-tagged nanoparticles.

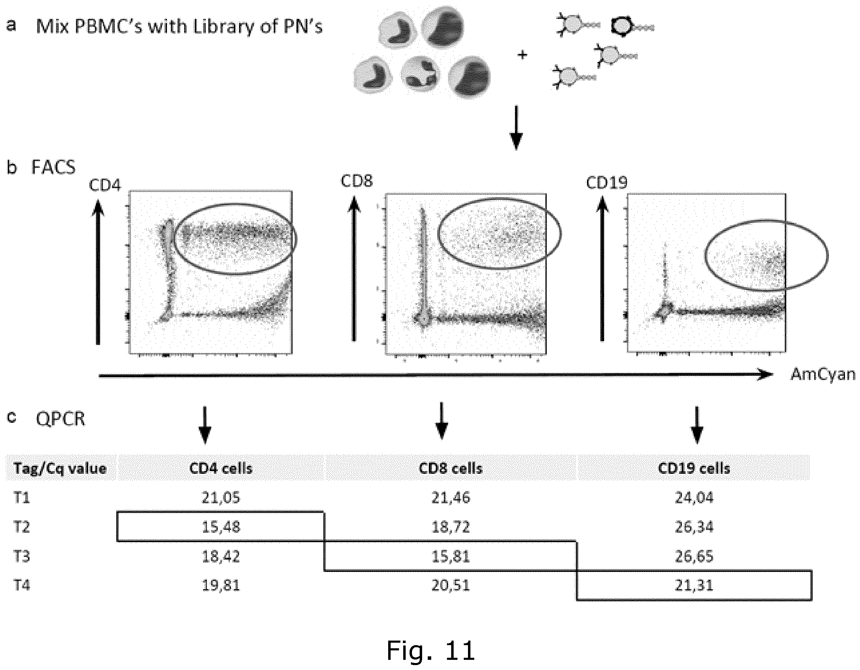

Figure 11 . Incubation of a library of DNA-tagged polystyrene (AmCyan filled) nanoparticles (PN's) with PBMCs. (a) Incubation of PN's with PBMCs. (b) FACS sorting cells (in circle) according to presence of AmCyan (corresponding to presence of cell-associated NP's) and CD molecule displayed on cells. Live lymphocytes were gated. (c) QPCR analysis, using tag specific primer sets, of DNA-tags associated with each population of sorted cells.

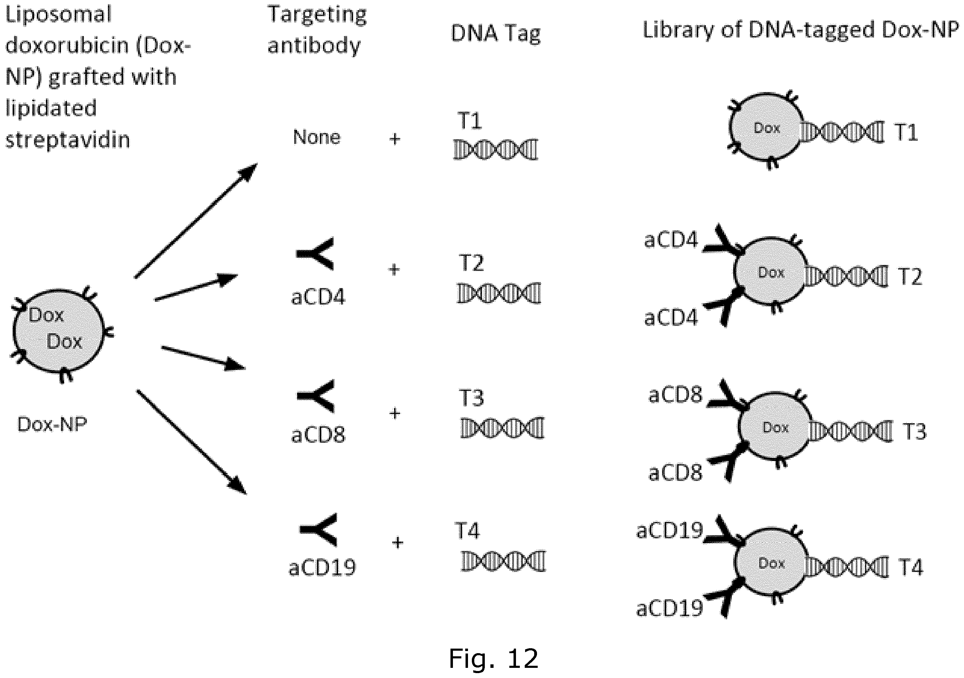

Figure 12: Synthesis of a 4-member DNA-tagged library of liposomes with doxorubicin (Dox) as cargo and targeting antibodies as surface molecules. First, doxorubicin loaded liposomes (Dox-NP) were grafted with lipid modified streptavidin. Streptavidin modified Dox-NP was then coated with biotinylated anti-CD4 antibody, anti-CD8 antibody, anti-CD19 antibody or no antibody respectively. Then, particles were linked to DNA-tags encoding the surface molecules. The result is a library of DNA-tagged targeted liposomes with doxorubicin cargo.

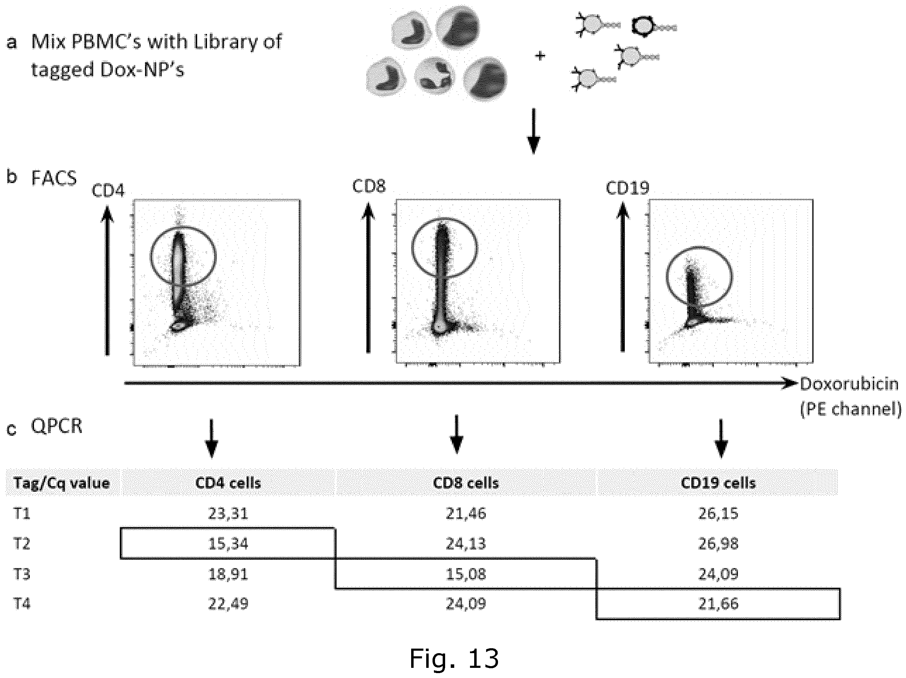

Figure 13: Incubation of a library of four DNA-tagged Dox-NP's with PBMCs. (a) Incubation of Dox-NP's with PBMCs. (b) FACS sorting of cells (in circles) according to CD molecule displayed on cells. Gating on live lymphocytes. (c) QPCR analysis, using tag specific primer sets, of DNA-tags associated with each population of sorted cells.

### Detailed description of the invention

An object of the present invention relates to the establishment of a method for producing nanoparticles suitable for identification after/during incubation in vitro, such as incubation with a target or a surface, after/during stress tests, such as accelerated stress tests, after/during incubation with a biological sample, such as a sample of primary cells or a cell cultures, or after/during incubation in vivo.

In one embodiment of the present invention is the method applied in vitro on for example blood samles or tissue suspensions.

In particular, it is an object of the present invention to provide a fast and reliable method for producing multicomponent nanoparticles encoded by a tag that solves the above mentioned problems of the prior art, including the current inability to efficiently synthesize and screen large libraries of different particle formulations, by providing a large library of particles from which single particles and their individual components can be identified by their unique tag in a high throughput manner, upon induction of an effect/change and/or upon displaying an effect/change.

The inventors have found that a method, in which individual components are assigned with unique tags upon synthesis, has proven to be a viable scheme for reliable synthesis and identification of single nanoparticles and their individual components in a large pool of, optionally partitioned, particles. Consequently, the present invention is ultimately suitable for high-throughput screening of large libraries of pharmaceutical drug formulations for efficacy and targeting *in vivo* and subsequent deconvolution of the influence of individual components of such a particle.

The present invention provides a versatile platform for development of new and innovative nanoparticles, because the characteristics of new components when present in particles can quickly be optimized through utilization of unique tags. Furthermore, the present invention provides a method for high throughput screening and optimization of existing pharmaceutical nanoparticles, and for targeted delivery of otherwise toxic drugs, which may be employed to salvage otherwise abandoned drug candidates.

From here onwards, several aspects and embodiments of the present invention will be described.

It should be noted that embodiments and features described in the context of one of the aspects of the present invention also apply to the other aspects of the invention.

### Particles

In the present invention, a particle is a nanometer to micrometer scale entity build from two or more of a variety of components and can have a range of sizes and shapes, e.g. shells or solids, spherical, elongated, cubic etc.

In the present invention, a component is a biological or synthetic entity which may be a ready-to-use building block or comprise two or more precursors that can be assembled or reacted to form a component. A component could be, but is not limited to, a carrier, a cargo, a surface molecule and a tag.

Consequently, a first aspect of the disclosure is a tagged particle comprising two or more components selected from the group consisting of:
i. a carrier,
ii. a cargo,
iii. a surface molecule, and
iv. a tag,
wherein at least one of the components is a tag.

Figure 1 exemplifies this aspect.

Another aspect of the present disclosure relates to a tagged particle comprising one or more components selected from the group consisting of a carrier, a cargo, a surface molecule, and furthermore a tag.

A preferred aspect of the present invention is a tagged particle comprising at least one component from each of a)-c):
a) a carrier (P) selected from the group consisting of liposomes, polymeric particles, micelles, albumin complexes, dendrimers, metal colloids and ceramics,
b) a cargo molecule (K) selected from the group consisting of drugs, oligonucleotides, proteins, antibodies, radioisotopes, markers, metal ions, adjuvants, organic molecules, small molecules and cytokines, and
c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
and an oligonucleotide tag comprising one oligonucleotide tag sub-segment (p, k, t, z) for each component from a)-c).

In another embodiment of the disclosure, the carrier (P) is selected from the group consisting of liposomes, polymeric particles, micelles, albumin complexes, dendrimers, metal colloids, ceramics, vectors such as viral vectors or leukolike vectors, vesicles such as proteolipid vesicles, cells such as targeted protocells, and cell mimics such as red blood cell mimics or PBMC mimics.

In yet another embodiment of the disclosure, the cargo molecule (K) is selected from the group consisting of drugs, oligonucleotides, proteins, antibodies, radioisotopes, markers, metal ions, adjuvants, organic molecules, small molecules, cytokines, peptides, antigens and lipids.

In a further embodiment of the disclosure, the surface molecule (Z, T) is selected from the group consisting of antibodies, antibody fragments, antibody mimics, peptides, proteins, ligands, aptamers, polymers, drugs, organic molecules, small molecules, sugars, oligonucleotides, carbohydrates, linkers, antigen-presenting molecules and MHC molecules.

In a preferred embodiment of the invention, a particle could comprise a cargo, *e.g.* a drug, encapsulated in a nanoscopic carrier, *e.g.* a liposome, to which are attached surface molecules to facilitate *in vivo* or *in vitro* stability and delivery, and a tag, e.g. an oligonucleotide, that allows identification and characterization of the tagged particle.

Thus, a preferred embodiment of the invention relates to a tagged particle as described herein, wherein
a) the carrier is a liposome,
b) the cargo is a drug, radioisotope or therapeutic oligonucleotide, and
c) the surface molecule is an antibody,
and the oligonucleotide tag is a DNA oligonucleotide tag comprising one DNA oligonucleotide tag sub-segment for each component from a)-c).

Furthermore, another preferred embodiment of the invention relates to the method as described herein, wherein:
a) the carrier is a liposome,
b) the cargo is a drug, radioisotope or therapeutic oligonucleotide, and
c) the surface molecule is an antibody,
and the oligonucleotide tag is a DNA oligonucleotide tag comprising one DNA oligonucleotide tag sub-segment for each component from a)-c).

### Tags

In the present invention, a tag is a biological or synthetic molecule attached to a particle, and can be comprised of minor building blocks that when combined create a code used to store information.

In one embodiment of the invention, the tag of the tagged particle comprises one tag sub-segment for each different or invidivual component.

A key feature of the present invention is the ability to tagged a particle according to component, features, concentrations etc. This means that any given particle can be identified by its tag or "barcode".

Thus, the tag comprises information about the particle. The information is selected from the group consisting of individual components, species of components, combinations of components, different components, concentrations, conditions and/or physical and chemical properties.

Any particle criteria mentioned herein can be stored as information in the tag.

In the present invention, a tag sub-segment is a region within a tag, allowing the tag to be comprised of two or more tag sub-segments. Each tag sub-segment may comprise a coding region, which allow identification of a single component or feature. Thus, a tag comprising two or more tag sub-segments may be used to identify individual components of a particle comprising two or more components.

In another embodiment of the invention, the tag of the tagged particle comprises one tag sub-segment for each different component or component precursor.

By tagging, and thereby encoding, individual components or their precursors, it is possible to identify the composition of very complex particles consisting of many components by decoding the corresponding tags.

The code of the tag can be chosen from a variety of encoding techniques that can store information based on a set of building blocks. One possibility is to encode the identity of individual components by a repeated sequence of a recognizable chemical structure.

In one embodiment of the disclosure, the tag of the tagged particle is selected from the group consisting of oligonucleotides and peptides.

Thus, an embodiment of the invention relates to the method (or tagged particle) as described herein, wherein the oligonucleotide tag comprises one oligonucleotide tag sub-segment for each different component or component precursor.

In another preferred embodiment of the invention, the tag of the tagged particle comprises polymers selected from the group consisting of DNA, RNA, LNA and PNA. Tags may also comprise derivatives or mimics of DNA, RNA, LNA, and PNA.

Another embodiment of the present invention relates to the method (or tagged particle) as described herein, wherein the oligonucleotide tag comprises polymers selected from the group consisting of DNA, RNA, LNA and PNA or derivates or mimics thereof.

A preferred embodiment of the invention relates to the method (or tagged particle) as described herein, wherein the oligonucleotide tag is a DNA oligonucleotide tag.

The four building blocks of nucleic acids provide a versatile platform for tagging/encoding many different components in an efficient manner. Furthermore, nucleic-acid-based tags may be easily amplified and sequenced by established methods.

The tag in its simplest form consists only of a coding region that is used to identify one or more individual components. By introduction of supplementary regions in the tag, additional functionalities can be introduced, such as primer annealing regions, probe sites, such as QPCR probe sites, random sequence regions to uniquely identify an individual tag among a multitude of tags, such as a multitude derived from PCR amplification.

In one embodiment of the invention, the tag or tag sub-segments of the tagged particle comprises:
i. a primer region,
ii. optionally an overlap region, and
iii. a coding region.

The introduction of a primer annealing regions facilitates amplification of oligonucleotide tags e.g. prior to sequencing.

A preferred embodiment of the present invention relates to the method (or tagged particle) as described herein, wherein the oligonucleotide tag comprises three or more oligonucleotide tag sub-segments each comprising:
i. an overlap region that facilitate hybridization of oligonucleotide tag sub-segments, and
ii. a coding region that encode a single component or component precursor,
and wherein one of the oligonucleotide tag sub-segments furthermore comprise a primer region that facilitate amplification of the oligonucleotide tag.

An important consideration when quantifying amplified oligonucleotide material is whether the resulting amount of oligonucleotide material originates from a single oligonucleotide source or from several identical oligonucleotide sources. To overcome this problem, small regions called unique molecular identifiers (UMI) may be added to the oligonucleotide material. These UMI regions are random sequences that subsequent to amplification may be used to determine the initial number of oligonucleotide sources of a specific sequence.

Thus, an embodiment of the present invention relates to the method (or tagged particle) as desctribed herein, wherein the oligonucleotide tag further comprises a unique molecular identifier (UMI) region of random nucleotide bases. Such a UMI region may contain 2-4, 4-6, 6-8 or 8-10 nucleotide bases. For very large libraries the UMI region may also contain more than 10 nucleotide bases.

A preferred embodiment of the present invention relates to the method (or tagged particle) as described herein, wherein the UMI region contain 6-8 random nucleotide bases.

When comprising polymers, such as polynucleotides, tags may be provided in single-stranded or double-stranded form.

Prior to/during/after synthesis of tagged particles, tags may be covalently joined by a number of methods, e.g. enzymatic ligation, chemical ligation, click chemistry, chemical transformations, such as amide bond formation or click chemistry.

When covalently joining tags they may be in single-stranded or double-stranded form. If in double-stranded form, tags may include overhang regions that facilitate annealing of tags to allow efficient covalent joining. Overhang regions may be provided as one or more single-stranded part of an otherwise double-stranded tag - or may be provided by a single-stranded 'splint' which can anneal to two single-stranded tag regions and position them for covalent joining.

DNA fragments with appropriate termini that are aligned on a complementary strand so that their ends are juxtaposed can be joined by DNA ligase to form a phosphodiester bond.

Thus, in one embodiment of the invention, the oligonucleotide tag, such as a DNA tag, is 5' phosphorylated to facilitate ligation of two or more tag subsegment.

An integral part of the invention revolves around the ability to recover and subsequently decode the unique tag of a particle. In order to accomplish this it is important that tags remain intact throughout the course of the screening, and do not interfere with delivery and/or celluar interaction.

Consequently, in one embodiment of the invention, inhibitors of tag degrading entities may be administered before, during, or after contacting the particle with the sample. Inhibitors may be, but are not limited to, enzyme inhibitors, ligands, receptors, protease inhibitors, nuclease inhibitors, phosphatase inhibitors, DNAse inhibitors, RNAse inhibitors, ligase inhibitors and combinations thereof.

In another embodiment of the invention, tags are protected by protecting groups and/or are encapsulated. This serves to protect the tags from degradation, e.g. in the bloodstream or inside a cell, such as a mammalian cell.

Another possibility is to encode the identity of single components in a mannar that allows visual decoding or decoding utilizing radiation, including electromagnetic radiation. Such decoding can be advantageous in some situations because the identification of the tag can be executed in a non-invasive manner.

In one embodiment of the disclosure, the code of the tag could therefore be based on fluorescence.

In another embodiment of the disclosure, the code of the tag could be based on digital molecular barcoding, e.g. a technique that is commercially available from NanoString Technologies Inc.

The digital molecular barcoding technique function as a unique tag comprised of a set of color-coded probes pre-mixed with a set of system controls. The setup enables the simultaneous utilization of a plethora of tags that can comprise one or more uniquely color-coded tag sub-segments, each representing an individual component. Hence, particles tagged with this system can be characterized by counting their fluorescent barcodes.

Independent of the nature of the tag it may be attached to the particle by a variety of methods, e.g. covalent coupling, adsorption or encapsulation.

In a preferred embodiment of the invention, the tag sub-segments are not physically connected to the components they encode. In this embodiment, the tag may be comprising two or more tag sub-segments joined by hybridization of an overlap region within each sub-segment.

Therefore, one embodiment of the present invention relates to the method (or tagged particle) as described herein, wherein the oligonucleotide tag sub-segments are not physically connected to the component or component precursor they encode.

In a further preferred embodiment of the invention, the tag comprising two or more tag sub-segments not physically connected to the components they encode is attached to the particle via covalently coupling of one tag sub-segment to the carrier component or carrier precursor.

Thus, an embodiment of the present invention relates to the method (or tagged particle) as described herein, wherein the oligonucleotide tag is attached to the particle via covalently coupling of one tag sub-segment to the carrier or carrier precursor.

There exist many possible scenarios, in which a particle and its associated tag upon contact with a sample will be located at an inaccessible location, e.g. within a cell.

In a preferred embodiment, more than one tag comprises an anchor, such as a lipid or polymeric anchor, such as a PEG anchor, to anchor one or more tags to a carrier/particle.

An anchor may be present at one end of a tag, e.g. at the 5' or 3' end of an oligonucleotide. Alternatively, an anchor may be attached internally in a tag. It may be attached to any part of a nucleotide, e.g. sugar, base or phosphate.

One or more tags may have an anchor and a tag may have more than one anchor, e.g. two or more same or different anchors.

A tag may have one or more other moieties attached, such as a small molecule, organic molecule, polymer, lipid, protein, antibody, antibody fragment, antibody mimic, biotin, another nucleotide.

Thus, in one embodiment of the invention, the sample is, subsequent to contact with a particle, denatured to release the tag from containment and allow decoding of the tag. Denaturation may be achieved by heating or use of denaturing agents, e.g. detergents and chaotropic salts.

Particle components may also be encoded by two or more seperate tags. In one embodiment of the disclosure, a particle has one tag which encodes the cargo and a separate tag which encodes one or more other carrier components. In another embodiment, a particle has one tag which encodes the surface molecule and a separate tag which encodes one or more other carrier components. For example a phage particle attached to a carrier, e.g. by making one or more chemical links between the phage particle and one or more carrier or by binding one or more phage particles to one or more carriers displaying anti-phage antibodies.

In another embodiment of the disclosure, a particle has one tag which encodes the cargo, another tag which encodes the surface molecule and a separate tag which encodes one or more other carrier components. For example, one or more DNA-encoded small molecule library compounds may be linked to one or more phage particles which encodes and displays one or more surface molecule(s), and one or more compounds and/or phage particles may be linked to one or more carriers.

### Particle properties

A particle will display a certain set of physical and chemical properties depending on factors in the surrounding environment it is located in. For example, the pressure and pH value of the solution the particles are immersed in may influence particle shape and charge. However, the particle properties can also be modulated by choosing specific combinations of components.

Thus, in one embodiment of the invention, the choice of carrier, cargo and/or surface molecule provides the particle with specific physical and/or chemical properties. Well-chosen combinations of components therefore allow the production of a wide variety of specialized particles with tailor-made properties such as enhanced stability, pH sensitive release modes, or sizes optimized for the enhanced permeability and retention (EPR) effect.

Therefore, in one embodiment of the invention, the properties of the particle is selected from the group consisting of size, morphology/shape, and stability, electrokinetic potential, chemical composition, charge, hydrophilic or hydrophobic properties, shape such as cubic, spherical, cylindrical, rod, disk, ring, rigidity and flexibility, stimuli-responsiveness, such as response to pH, light or other radiation, proteases, temperature, porosity, such as the fraction of shape which can be occupied by cargo absorption, such as absorption rate from intestines distribution, such as distribution rate in a tumor, blood vessel, metabolic rate, such as rate of modification or degradation, excretion rate, toxicological properties immunological properties, such as binding to immune system cells or proteins, dissasembly rate, such as the dissasebly rate of a complex of protein particles, such as albumin particles in a biological fluid such as blood level of covalent or noncovalent crosslinking, content of water, solvent or buffer, degree of swelling in a solvent or buffer, largest diameter, or smallest diameter.

The large amount of available and diverse components makes it possible to design a plethora of particles, all with distinct physical and chemical properties. The strength of the present invention is the ability not only to generate these diverse particles in an organized manner, but also have the ability to sort them based on their efficiency in an unprecedented systematic fashion. By keeping track of large amounts of particles based on their efficiency or other characteristics, it becomes possible to map patterns and pick components that work well together and ultimately generate particles with synergetic properties and superior effects.

### Carrier

In the present invention, a carrier is a physical entity used to protect and transport a cargo to its destination of delivery. A carrier can be of biological or synthetic origin and the choice of carrier largely depends on the purpose of the particle.

In one embodiment of the invention, the carrier component of the particle is selected from the group consisting of liposomes, polymeric particles, micelles, albumin complexes, dendrimers, metal colloids and ceramics.

In embodiments of the present disclosure, the carrier comprises polymers, such as lipids, copolymers, proteins, peptides, oligonucleotides. The carrier may also comprise salts, cofactors, etc.

An embodiment of the present invention relates to the method (or tagged particle) as described herein, wherein the carrier is a liposome or a polymeric particle.

In one embodiment of the invention, the carrier component of the particle is a liposome.

Liposomal carriers have been utilized as carriers of active ingredients in a plethora of scientific studies, but only a minor fraction of the efforts have resulted in successfully FDA approved products, e.g. Doxil, Myocet, AmBisome and Marqibo. With the screening platform disclosed by the present invention the rate of success may be significantly increased due to the ability of conducting systematic high throughput screening.

In one embodiment, carriers are loaded with cargo from a compound collection, e.g. using robotics. For example empty carriers are organized in wells and to each well a different compound is added, such as 1-0, 10-100, 100-1000 different compounds/wells. Cargo is allowed to diffuse into particles, the particles are tagged, and unlinked tags and free compound is removed, and particles are combined to form a library.

### Cargo

In the present invention, a cargo may be a molecule or collection of atoms. Cargo can as a non-limiting example be connected to the carrier by encapsulation or intercalation.

Consequently, in one embodiment of the invention, the cargo of the particle is selected from the group consisting of drugs, oligonucleotides, proteins, antibodies, radioisotopes, markers, metal ions, adjuvants, organic molecules, small molecules and cytokines.

In another embodiment of the invention, the cargo of the particle can be, but are not limited to, the group consisting of DNA damaging agents, cytostatic agents, agents that inhibit cancer cell invasion, inhibitors of growth factor function, antiangiogenic agents, vessel damaging agents, antisense therapeutic agents, gene therapeutic agents, immunotherapeutic agents, and combinations thereof.

Cargo can also be chosen to deliver genetic information, e.g. in the form of DNA or RNA, to a cell. A typical manifistation of said genetic information could be oligonucleotides in the form of vectors. In a preferred embodiment, the cargo may comprise DNA or RNA encoding sequences in a format that allow transcription upon transfer to a given cell type, e.g. transfer of T-cell receptor gene sequences to CD8 T cell in vivo, avoiding the laborious vitro manipulation currently need for gene transfer to specific cell-types. The cargo of the tagged particle may thus be intended for gene delivery and/or gene therapy.

A preferred embodiment relates to the method (or tagged particle) as described herein, wherein the cargo is a selected from the group consisting of a drug, a radioisotope and a therapeutic oligonucleotide.

In another embodiment of the invention, cargo may be targeted for distribution to specific biological tissue/anatomical sites, in which case the cargo could be e.g. an immune stimulatory molecule, such as a cytokine, adjuvant, immune regulatory molecules of antibodies affecting immunological pathways. The cargo could be preferentially delivery to a given tissue, e.g. tumor lessions or lymph nodes.

The wide range of potential cargo molecules enable many possible uses of the particles, ranging from pharmaceutical formulations comprising cytotoxic drugs against specific diseases, to particles carrying contrast agents to specific tissues for imaging techniques such as CT and MRI scans.

It can be advantageous to supply the above mentioned types of cargo in a particle as described in the present invention to protect the cargo from clearance upon entry in a subject. Technologies based on encapsulation of an active ingredient are already employed within many industries, but optimization of such formulations remains a daunting and expensive challenge due to the many possible component combinations. With the present invention it is possible to categorize the type of particle most suitable for a given active ingredient and thereby not only facilitate development of new products but also enable salvaging of drugs abandoned due to challenges with pharmacodynamics or pharmacokinetics.

Most DNA-encoded libraries comprise a DNA "barcode" linked to a single small molecule or macrocycle, synthesized from a single chemical handle (often a primary) amine present on the piece of DNA used in the first step(s) of synthesis. If instead multiple chemical handles are used, e.g. as present on a linear or branched polymer, such as a dendrimer, it is possible to synthesize a large number of compounds linked to one or more DNA tags so that each compound is identical and each DNA tag is also identical. Said number of compounds may be 1-10, 10-100, 100-1000, 1000-10000, 10000-100000, 100000-1000000, 1000000-10000000, 10000000-100000000, 100000000-1000000000 or more.

An example of a scaffold with multiple chemical handles, such as multiple amino groups is PAMAM. PAMAMs and other scaffolds with multiple chemical handles may be used as starting points for DNA-encoded small molecule synthesis.

DNA-encoded cargo molecules, e.g. covalently linked to a linear or branched polymer, may be wrapped in a liposome or a polymeric nanoparticle. For example, dendrimers may be wrapped by/locked-in by liposomes for cancer therapy.

### Surface molecule

In the present invention, a surface molecule is a biological or synthetic molecule attached to the particle.

In one embodiment of the invention, the surface molecule component of the particle is selected from the group consisting of antibodies, antibody fragments, antibody mimics, peptides, proteins, ligands, aptamers, polymers, drugs, organic molecules, small molecules, sugars, oligonucleotides, carbohydrates and linkers.

A surface molecule may facilitate the recognition of a specific target or enhance the stability of the particle in the sample, e.g. specific cell targeting can be realized by utilization of antibody surface molecules and increased particle circulation half-lives can be achieved by customization of particles with polyethylene-glycol.

A preferred embodiment of the invention relates to the method (or tagged particle) as described herein, wherein the surface molecule is an antibody.

In one preferred embodiment of the invention, the surface molecules are antibodies against CD8, CD19 or a combination thereof.

Another embodiment of the invention relates to the method (or tagged particle) as described herein, wherein the surface molecule is an antibody against CD8 or CD19 or a combination thereof.

In one embodiment of the invention, surface molecules are molecules which can bind to one or more components of a plasma membrane, such as A33, Angiopoietin 1, Angiopoietin 2, CAIX, CC49, CD19, CD20, CD30, CD33, CD52, CEA, CTLA4, EGFR, EpCAM, EPHA3, ERBB2, ERBB3, FAP, Folate-binding protein, GM2, gp100, gpA33, IGF1R, Integrin α5β1, Integrin αVβ3, Lewis antigen Y, MET, Mucins, PSMA, RANKL, TAG-72, Target, Tenascin, TRAILR1, TRAILR2, VEGF, VEGFR.

In one embodiment of the invention, surface molecules are molecules which can bind to one or more components of endosomes such as EEA1, Rab5, Rab7, Rab7, Rab11, Pallidin.

In one embodiment of the invention, surface molecules are molecules which can bind to one or more components of lysosomes such as LC3, LAMP2, ATG5.

A preferred embodiment of the invention relates to the method (or tagged particle) as described herein, wherein the surface molecule is capable of binding one or more molecules located on an entity selected from a plasma membrane, an endosome and a lysosome.

In one embodiment of the invention, the surface molecules may bind any surface presented by biological components, e.g. in vitro, such as a purified protein, a protein, cell membrane or cell membrane consituents, an organ, an organelle, an anatomical surface or site, for example tumor stroma or tumor vasculature.

Targets for surface molecules may be present on cells, in cells, or released from cells. It may also be targets in solutions such as albumin or other another component in an organism. Targets also include components introduced into a body, such as a stent or tube.

Targets may also be non-biological surfaces such as metal, glass, plastic or ceramic.

In a preferred embodiment, one or more surface molecules are chosen from cell penetrating compounds, such as cell-penetrating peptides.

Targeting of CD19 or CD20 (or combinations hereof) would be of preferred interest in B-cell malignancies. This patient group is today subjected to harsh chemotherapeutic regiments until observation of dose-limiting toxicity. Targeted delivery of chemotherapeutic drugs or drug combinations to B-cells could potentially greatly enhance treatment efficacy and reduce side effects for this patient group.

Targeting of CD8 T cells, is of particular interest to supply certain receptor recognition elements to CD8 T cell populations in vivo, e.g. through transfer of T cell receptor genes or chimeric-antigen-receptor (CAR) constructs.

In another preffered embodiment, the surface molecules of the particle can be antibodies against neo-vasularisation markes frequently upregulated in both cancer and inflammatory diseases. Neo-vascularisation/angiogenesis is a characteristic feature of virtually all aggressive tumors and of several non-oncological conditions, including rheumatoid arthritis, psoriasis, vasculopathy, blinding ocular disorders, atherosclerosis, inflammatory bowel diseases and endometriosis. Specific molecules described as preferential target are fibronection and Tenacin-C.These molecules have already been exploited for specific delivery of drugs to malignant and inflamed tissues. With the present invention we can potentially greatly enhance the efficacy of such delivery systems through high-through evaluation of vast numbers of combinations of particle, cargo and surface molecules.

In one embodiment of the invention, the surface molecule is provided as part of a display entity. For example, a surface molecule may be chosen from a peptide displayed on a phage particle, an antibody displayed on a phage particle, or a peptide displayed on a ribosome, such as ribosome display.

In another embodiment of the invention, a display entity may be linked to a carrier, such as a liposome or nanoparticle, wich comprise cargo that can induce an effect/change in a sample.

In yet another embodiment of the invention, members of a phage display library may each be linked to a carrier thus forming a new library of carrier-linked phage particles displaying peptide(s). If the same carrier is linked to the phage particles, the library can be used - by incubating with a sample followed by analysis acoording to the method of the present invention - to identify peptides with desired characteristics such as the ability to efficiently cause the linked carrier to induce an effect/change in a sample.

### Component precursors

Many types of components are aggregates formed from smaller molecular units that in solution come together to form a superstructure. Consequently, a component may be comprised of one or more component precursors, e.g. different lipid species in liposomes or different polymers, such as copolymers, in polymeric particles. Different parts of an antibody may also be regarded as component precursors, *e*.*g*. light chain, heavy chain, Fab region or Fc region.

In an embodiment of the invention, one or more components may be formed from component precursors. Thus, another embodiment of the invention relates to the method (or tagged particle) as described herein, wherein one or more components are formed from two or more component precursors.

For each component precursor an oligonucleotide tag sub-segment is added so that it is possible to identify also components by their parts. Thus, it is attainable not only to identify tagged particles by their components but also by their component precursors.

### Synthesis of tagged particles

In addition to disclosing particles comprising a set of components, each uniquely tagged for easy identification and characterization of each individual particle, the present invention also provides a simple and efficient method for producing a multitude of such particles.

Therefore, a second aspect of the disclosure is a method for the production of a plurality of tagged particles, the method comprising at least the step of:
i. mixing at least two components selected from the group consisting of:
   a. a carrier,
   b. a cargo,
   c. a surface molecule, and
   d. a tag,
wherein at least one of the components is a tag, thereby forming a plurality of tagged particles.

See figure 3, which exemplifies this aspect.

### Mix and split synthesis

An easy way to synthesize a large library of different particles, while still keeping track of each single combination of particle components, is by a mix and split synthesis. This type of synthesis is often used within combinatorial chemistry to produce large libraries of peptides or small molecules.

Thus, a preferred second aspect of the present invention is a split and mix method for the production of a plurality of tagged particles, the method comprising at least the step of:
i. mixing one component from either of a)-c):
   a) a carrier (P) selected from the group consisting of liposomes, polymeric particles, micelles, albumin complexes, dendrimers, metal colloids and ceramics,
   b) a cargo molecule (K) selected from the group consisting of drugs, oligonucleotides, proteins, antibodies, radioisotopes, markers, metal ions, adjuvants, organic molecules, small molecules and cytokines, and
   c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
   with an oligonucleotide tag sub-segment (p, k, t, z) to form a first solution of first generation tagged particles,
ii. splitting said first solution of first generation tagged particles into two or more first solutions of first generation tagged particles,
iii. mixing said two or more first solutions of first generation tagged particles with one component from either of a)-c) and an oligonucleotide tag sub-segment (p, k, t, z) to form two or more second solutions of second generation tagged particles,
iv. splitting said second solution of second generation tagged particles into two or more second solutions of second generation tagged particles,
v. mixing said two or more second solutions of second generation tagged particles with one component from either of a)-c) and an oligonucleotide tag sub-segment (p, k, t, z) to form two or more third solutions of third generation tagged particles,
and wherein the plurality of tagged particles resulting from step v) comprise at least one component from each of a)-c).

It is to be understood that the term "solution" describes all types of liquid solutions containing particles, such as dispersions, suspensions and others.

Therefore, one embodiment of the disclosure, is the above method wherein step i) is characterized in that one component is mixed with a tag sub-segment, forming a solution of first generation tagged particles.

In another embodiment, said solution of first generation tagged particles is split into two or more solutions of first generation tagged particles.

In a further embodiment, said two or more solutions of first generation tagged particles is mixed with one component selected from the group of components given in step i in the above method and a tag sub-segment, forming two or more solutions of second generation tagged particles.

In a preferred embodiment the above procedure is repeated one or more times to form further generations of tagged particles.

Another embodiment of the invention relates to the split and mix method as described herein, wherein steps iv)-v) are repeated one or more times to form further generations of tagged particles.

One embodiment of the invention relates to the split and mix method as described herein, wherein the particle components are assembled from two or more component precursors that each are encoded by an oligonucleotide tag sub-segment.

Utilizing this for synthesis of a multitude of particles, it is possible to generate libraries containing millions of unique particles.

Therefore, one embodiment of the invention relates to the split and mix method as described herein, wherein the plurality of tagged particles contain at least 1000 unique particles.

### Purification of particles

During synthesis it may be necessary or advantageous to purify successive generations of particles. The purification could be focused at separation of particles from non-reacted components in solution or at separation of different particles from each other.

In one embodiment of the invention, the plurality of tagged particles synthesized by the above described method are purified by a technique selected from the group consisting of sedimentation, size exclusion chromatography, centrifugation or filtration.

### Precursors to components

As previously described, the components of a particle may be formed by component precursors. The mix and split synthesis is suitable for also including component precursors and incorporate their identity into the unique tag of the particle.

Thus, in one embodiment of the invention, the particle components are assembled from two or more component precursors. For example, a tag may be formed by two or more component precursors each comprising a tag sub-segment.
In a preferred embodiment, cargo is assembled from precursors, e.g. by a split and mix method. Cargo may be assembled before, during, or after the formation of a particle. For example, 3 first cargo precursors are reacted in a mix and split fashion with 3 second cargo precursors, and then in a mix and split fashion with 3 third cargo precursors, to form a total of 3 x 3 x 3 = 27 cargo molecules. By performing such assembly of cargo molecules in parallel or in series with particle assembly, particles with unique cargo or a combination of cargo molecules may be formed.

The inclusion of particle component precursors in the method of synthesis will further diversify the resulting library of particles and increase the amount of unique particles by several orders of magnitude. This can be exemplified by the inclusion of for instance five lipid species and combination of those for formation of a liposomal carrier.

In one embodiment of the invention, one or more carrier components are synthesized by a split-and-mix method as known in chemistry, such as medicinal chemistry and/or polymer chemistry.

In another embodiment of the invention, cargo is provided by DNA-encoded split-and-mix library synthesis (see e.g. example 10).

### Particle library

The mix and split synthesis allow the generation of large libraries of peptides, small molecules, or as in present invention, complex particles. However, only a few examples exist, in which all entries of a particle library can be screened simultaneously. Known methods typically enable screening in the range of 100 particles in a single experiment.

The present invention may be used to improve particle screening capability by a factor of tens to thousands to millions.

Therefore, in one embodiment of the invention, the plurality of tagged particles contain at least 1000 unique particles.

The ability to screen a large number of particles in a high throughput manner represents a significant advantage of the present invention over known methods.

### Identification of individual components of tagged particles

An integral part of the present invention is the ability to partition/recover, identify, and characterize particles of interest subsequent to contact with a sample.

Thus, a third aspect of the disclosure is a method for the identification of individual components of a particle, the method comprising the steps of:
i. providing a plurality of tagged particles comprising two or more components selected from the group consisting of:
   a. a carrier,
   b. a cargo,
   c. a surface molecule, and
   d. a tag,
   wherein at least one of the components is a tag,
ii. contacting said plurality of tagged particles with a sample,
iii. evaluating the effect of the tagged particles on the sample or the effect of the sample on the tagged particles,
iv. identifying one or more tagged particles displaying and/or causing a specific effect,
v. recovering from the sample said one or more tagged particles displaying and/or causing the specific effect, and
vi. identifying by their tag individual components of said one or more recovered tagged particles.

The method differentiate itself from known technologies, in that the particles may be screened for efficacy/efficiency upon contacting a sample instead of simply affinity screening. By evaluating the particles both in vitro and in a biological sample e.g. in vivo, it is possible to derive information for clinical translation.

In an embodiment of the invention, the method is for the identification of tagged (multicomponent) particles with the ability to induce a biological, morphological, chemical, biochemical, catalytic, physical and/or physiological changes on the sample.

In another embodiment of the invention, the method is for identification of individual components of a tagged (multicomponent) particle with the ability to induce a biological, morphological, chemical, biochemical, catalytic, physical and/or physiological changes on the sample.

A preferred aspect of the present invention is a method for the identification of individual components of a particle, the method comprising the steps of:
i. providing a plurality of tagged particles comprising at least one component from each of a)-c):
   a) a carrier (P) selected from the group consisting of liposomes, polymeric particles, micelles, albumin complexes, dendrimers, metal colloids and ceramics,
   b) a cargo molecule (K) selected from the group consisting of drugs, oligonucleotides, proteins, antibodies, radioisotopes, markers, metal ions, adjuvants, organic molecules, small molecules and cytokines, and
   c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
   and an oligonucleotide tag comprising one oligonucleotide tag sub-segment (p, k, t, z) for each component from a)-c),
ii. contacting said plurality of tagged particles with a sample,
iii. evaluating the ability of the tagged particles to induce biological, morphological, chemical, biochemical, catalytic, physical and/or physiological changes on the sample,
iv. identifying one or more tagged particles causing a specific change,
v. recovering from the sample said one or more tagged particles causing the specific change, and
vi. identifying by their oligonucleotide tag the at least one component from each of a)-c) of said one or more recovered tagged particles.

In one embodiment of the present invention, step ii occurs *in vitro* or *in vivo.*

In another embodiment of the invention, a library of particles may be synthesized by using an encoded library of surface molecules and an encoded library of cargo molecules.

### Sample

In the present invention, a sample may be chosen from any biological solution, entity or subject. This can include, but is not limited to, organisms, biological fluids, tissues, organs and metastases, e.g. humans or mammals and all of their body parts. The sample may be a specific cell type within an organism, e.g. a cell overexpressing a protein or receptor.

Thus, an embodiment of the invention relates to the method as described herein, wherein the sample is selected from the group consisting of organisms, biological fluids, tissues, organs, cells and metastases.

Therefore, in one embodiment of the invention, the sample of step ii is an *in vivo* sample.

In another embodiment of the invention, the sample is an organism selected from, but limited to, the group consisting of a mammal, a primate or a human, preferably a human.

An embodiment of the invention relates to the method as described herein, wherein the organism is selected from the group consisting of a mammal, a primate or a human, preferably a human.

In a further embodiment of the invention, the sample is selected from, but not limited to, the group consisting of an organ, a tissue, a tumor, a bacteria or a virus, a cell or biological fluid.

Biological fluids include, but are not limited to, the group consisting of plasma, blood, saliva, urine, semen, vaginal fluid, sweat and serum.

An embodiment of the invention relates to the method as described herein, wherein the biological fluid is selected from the group consisting of plasma, blood, saliva, urine, semen, vaginal fluid, sweat and serum.

Cells can be human or animal cells and include, but are not limited to, the group consisting of diseased cells, cancer cells, primary cells, stem cells and immune cells.

An embodiment of the invention relates to the method as described herein, wherein the cell is selected from the group consisting of diseased cells, cancer cells, primary cells, stem cells and immune cells.

A sample may also be an in vitro sample, such as purified protein(s).

### The effect/change of - or on - the particle

In the present context, the two terms "effect" and "change" will be used interchangeably.

When a particle is brought into contact with a sample, two overall effects/changes may be observed. A change to the sample caused by the particle and/or a change to the particle caused by the sample.

Thus, in one embodiment of the invention, the effect/change of the tagged particles on the sample of step iii is evaluated by a criterion selected from the group consisting of the ability of the tagged particle to induce biological, morphological, chemical, biochemical, catalytic, physical and/or physiological changes on the sample.

In another embodiment of the invention, the effect/change of the sample on the tagged particles of step iii is evaluated by a criterion selected from morphological, chemical or physical changes to the particle.

Each of these effects/changes can be divided into one or a combination of specific effects/changes related to the corresponding effect/change on the sample. Specific effects/changes can be biological indicators associated to certain types of disease and may therefore be desirable readout for screening of particles. Identifying particles with the ability to enhance or reduce certain specific effects/changes is a central focus of the present invention.

In one embodiment of the invention, the specific effect/change of the tagged particles on the sample of step iv is selected from the group consisting of particle localization, cell apoptosis, cell cycle arrest, cell necrosis, cell proliferation, chemotaxis, multidrug resistance, signal transduction, protein expression and gene expression.

In another embodiment of the invention, the specific change induced by the tagged particles on the sample of step iv) is selected from the group consisting of, cell apoptosis, cell cycle arrest, cell necrosis, cell proliferation, chemotaxis, multidrug resistance, signal transduction, protein expression, gene expression and antigen presentation.

Effects/changes on the particle exerted by the sample may be equally important. Hence, they present invention may be used to test the integrity of particles with desirable pharmaceutical characteristics when stored in a variety of conditions, *e.g.* in blood, in plasma or in other biological fluids.

Thus, in another embodiment of the invention, the specific effect/change of the sample on the tagged particles of step iv is selected from the group consisting of changes in particle size, stability and electrokinetic potential.

Furthermore, it is important that particles with desirable characteristics maintain their chemical and physical properties when stored over periods of time. The present invention allow screening of particles for their compatibility with preferable storage options such as freeze-drying or as a solution.

### Sensitization

In one embodiment of the invention, the sample is sensitized to improve the detection level. For example, a cell line may be manipulated to show effects/changes of very low concentrations of molecules deliverd by a carrier, such as particles or liposomes. For example, cells may be manipulated to carry reporter systems to detect the effect of very few molecules present in the cell, such as 1-10, 10-100, 100-1000, 1000-10000, 10000-100000, 100000-1000000, 1000000-10000000, 10000000-100000000, or 100000000-1000000000 molecules.

Cells may be sensitized e.g by adding a sensitizer to all particles, by adding a sensitizer to the cell medium, or by radiation of the cells. A sensitizer may thus be *e.g.* a component of a combination therapy or a compound with limited toxicity. A single or a combination of sensitizers may be used to block all but one of known redundant cellular pathways, so that the cell becomes sensitive to modulators of the remaining pathway.

### Readout

In the present invention, the readout refers to the measureable quantity that reports on the occurrence of a specific effect/change as described previously.

As an example, many of the cellular processes designated as specific effects/changes above lead to changes in intracellular radicals (including Nitric Oxide), free-ion concentrations (Ca2+, Mg2+, Zn2+ and other metal ions), pH, Na+, K+, CI- and miscellaneous ions or membrane potential that can be followed with appropriately responsive fluorescent indicators.

Furthermore, decoding of particles carrying contrast agents may involve imaging techniques for identification.

For assessment of the effect/change of the sample on the particle, important parameters include size, stability and electrokinetic potential.

Thus, in one embodiment of the invention, the specific effect/change of step iv is identified by a readout selected from the group consisting of fluorescence imaging, nuclear imaging, radioactive imaging, X-ray imaging, dynamic light scattering, electrophoretic mobility or a combination thereof.

In some cases, it may be preferable to use two or more complementary techniques to identify a single specific effect/change, e.g. a combination of assays to measure enzymatic activity, membrane permeability, cell surface markers and redox potential.

One approach to assessing the stability of a particle is by measuring its size. Methods for determining particle size may be selected from, but are not limited to, the group consisting of dynamic light scattering, gel electrophoresis, rate zonal separation in viscosity gradient, sedimentation gradient centrifugation, flow cytometry sorting, size exclusion chromatography and combinations thereof.

The electrokinetic potential of a particle may be assessed by the electrophoretic mobility of that particle. The electrophoretic mobility is typically computed from zeta potential measurements or by gel electrophoresis techniques.

### Method for recovering particles of interest

After bringing the particles in contact with the sample and identifying the specific effect/change of interest, particles can be partitioned/recovered from the sample for analysis by a selection of methods.

Each type of specific effect/change will have a characteristic phenotype on which the evaluation of the efficiency of the particles may be based. As an example, apoptotic cells may be recovered by flow cytometry, magnetic cell sorting or a combination of these techniques.

Therefore, in one embodiment of the invention, the recovering of one or more tagged particles from the sample of step v is performed by a method of purification selected from the group consisting of fluorescence-activated cell sorting (FACS), flow cytometry, magnetic cell sorting, filtration, affinity chromatography, size exclusion chromatography, sedimentation, centrifugation, surgical excision or a combination thereof.

The recovery of tagged particles of interest may be performed with the sample being in solution or immobilized covalently or non-covalently to a solid support, *e.g.* a matrix, beads, a microtiter plate, a reagent tube or a chromatographic column.

### Decoding the tag

The final step in the screening for new efficient particles is the ability to identify every single component of the particle. The identification of single components is accomplished by decoding of the unique tag associated with every species of component. The choice of technique for decoding the tag depends on the nature of the tag.

In an embodiment of the invention, the identification of individual components of step vi is accomplished by one or a combination of techniques, the techniques being selected from the group consisting of PCR, RT-PCR, qPCR, sequencing, DNA microarray hybridization, fluorescence detection, mass spectrometry or a combination thereof.

The fragmentation of the particle into component supports a systematic concept, in which the development of new efficient particles are based on the performance of individual component and the interplay of different species of component.

### Use of multicomponent particles for therapy and diagnostics

Once particles with preferred characteristics and the ability to induce a desired effect/change has been identified, these particles may be used in a medical context.

The particles may for instance be as a medicament for treatment or amelioration of disease. Such diseases could be, but are not limited to, to the group consisting of cancer, neurodegenerative diseases, inflammatory diseases, chronic diseases, infectious diseases, mental diseases and genetic disorders.

Another possible use of the particles could be in diagnostics where contrast agents are necessary. The particles may find use in techniques such as CT scanning or MRI scanning.

Finally, the present invention may be used to screen for particles particularly suitable for a certain type of administration to a subject, e.g. oral, epidermal, inhalation or injection.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Example 1 (E1): Stability of tag on particle during sample contact

Example summaryThis is an example where the purpose is to check the stability of an oligonucleotide tag on a carrier following contact with a sample. The sample is peripheral blood mononuclear cells (PBMCs), a family of cells including lymphocytes, monocytes and macrophages, all of which are essential constituents of the immune system.

The carrier and cargo is in the form of a commercially available fluorescein-containing polymer bead (Spherotech, #SVFP-0552-5, USA, Illinois). The polystyrene beads are surface modified with streptavidin through which biotinylated DNA-oligonucleotide tags are bound to the carrier. No additional surface molecules are attached to the particle.

The particles are contacted with PBMCs but no separation step is performed. The integrity of DNA-oligonucleotide tags are analyzed by qPCR using tag-specific qPCR probes.

### Preparation of sample

### Collecting sample

Blood is obtained from the Danish blood bank (Dept. clinical immunology, 'Rigshopitalet', Denmark).

### Modifying sample

Peripheral whole blood is first separated at the blood bank to obtain a 'buffy coat', i.e. mixture of peripheral mononuclear cells (PBMCs), red blood cells and remaining plasma. PBMCs are isolated from whole blood by density gradient centrifugation. The density gradient medium, Lymphoprep (Axis-Shield), which consists of carbohydrate polymers and a dense iodine compound, facilitate separation of the individual constituents of blood. Blood samples are diluted 1:1 in RPMI (RPMI 1640, GlutaMAX, 25 mM Hepes; gibco-Life technologies) and carefully layered onto the Lymphoprep. After centrifugation, 30 min, 390 g, PBMCs together with platelets are harvested from the middle layer of cells. The isolated cells, the buffy coat (BC), is washed twice in RPMI and cryopreserved at -150°C in fetal calf serum (FCS; gibco-Life technologies) containing 10% dimethyl sulfoxide (DMSO; Sigma-Aldrich).

Prior to use, BCs are thawed in 10 ml, 37°C, RPMI with 10% fetal bovine serum (FBS), centrifuged 5 min, 1500 g, and washed in 10 ml RPMI with 10% FBS. All washing of cells refer to centrifugation 5 min, 490 g, with subsequent removal of supernatant. 10-20 million cells are washed in 400 µl barcode-buffer (PBS/0.5% BSA/2 mM EDTA/100 µg/ml herring DNA) and resuspended in this buffer to approximately 20 µl per sample.

### Production of tagged particles

### Synthesis

In this example, the tagged particle was a commercially available polystyrene bead (carrier) loaded with fluorescein (Yellow particles) (cargo) which is modified with attachment of streptavidin. The particle was tagged with a biotinylated oligonucleotide (tag) to encode the specificity of the antibody.
- ***Carrier*:** Streptavidin Coated Fluorescent Yellow Particles (carrier) (Spherotech, #SVFP-0552-5, USA, Illinois, 1 mg/ml, 400-600 nm).
- ***Cargo*:** Polystyrene beads are preloaded with fluorescein .
- ***Tag*:** Three oligonucleotide tags all containing 5' biotin, Code-1OS-1-Oligo-1, Code-1OS-1-Oligo-2 and Code-10S-1-Oligo-3 as described in Table 1 below were purchased from DNA-Technology.
- No surface molecules were attached to the particles

| Table 1 Sequence name | Sequence (5' --> 3') | Modification |
|---|---|---|
| Code-1OS-F1 | GGCTCCCGGATTTTGTAAGAATG (SEQ ID No:1) | |
| Code-1OS-R1 | GTTGTACCTAAGTAGAGACTGC (SEQ ID No:2) | |
| Code-1OS-1-Oligo-1 | | 5 = Biotin-C6 |
| Code-1OS-1-Oligo-2 | | 5 = Biotin-C6 |
| Code-1OS-1-Oligo-3 | | 5 = Biotin-C6 |
| A-Key Code-1OS-F1-1 | | |
| A-Key Code-1OS-F1-2 | | |
| P1-Key Code-1OS-R1 | | |
| Code-QProbe-1 | 2 CAG ATG GGA CTA CGC CAC CTC AAT X | 2 = FAM; X= BHQ-1 |
| | Tm=60,6 | |
| | (SEQ ID No:9) | |
| Code-QProbe-2 | 2 ATG CGT CTG CGT TGG TGA ATC GAT A X | 2 = CFR-610; X= BHQ-2 |
| | Tm=60,6 | |
| | (SEQ ID No:10) | |
| Code-QProbe-3 | 2 CAT CCT GCG TCT CGC ATA CCA GTT T X | 2= Qua670; X = BHQ2 |
| | Tm=61 | |
| | (SEQ ID No:11) | |

### Synthesis of tagged particles

In three individual preparations of particles the three biotinylated oligonucleotide tags were attached respectively to the particle surface via streptavidin/biotin linkage. Each particle was tagged with 2-10 oligonucleotide tags.

### Purification

Excess oligonucleotide tags was separated from particles by size-exclusion centrifugation (300kD, Pall Corporation, USA, OD300C34, or Sartorius, Germany, VS0151)

### Contacting tagged particles with sample

Amount of sample
10-20 million PBMCs were used

### Amount of tagged particle

100 million tagged particles per sample.

### Conditions during contact

Cells are resuspended in 20 µl barcode-buffer (PBS/0.5% BSA/2 mM EDTA/100 µg/ml herring DNA) per staining. Tagged particles are centrifuged for 5 min, 3300 g, prior to addition to cells. After adding tagged particles, the cells are incubated 30 min, 4°C. Following the mixtures was washed and subjected to additional 0, 30, 60 min incubation at 20°C.

### Recovering tagged particles causing specific effect/change

Apply assay for separation
Sample was not subjected to separation.

### Wash

No Washing

### Separate tagged particles

Not applied

### Identifying particle components by the unique particle tag

Secure tags on separated particles
Selected cells were stored at -80 °C.

### Apply method for deconvolution of tag information

The abundance of oligonucleotide tags, Code-1OS-1-Oligo-1, Code-1OS-1-Oligo-2 and Code-1OS-1-Oligo-3 was determined in the individual sorted fractions by quantitative PCR using the forward primer Code-1OS-F1 together with the reverse primer Code-1OS-R1 and the Code-1OS-1-Oligo-1, Code-1OS-1-Oligo-2 and Code-1OS-1-Oligo-3specific Q-PCR probes Code-QProbe-1, Code-QProbe-2 and Code-QProbe-3. For sequences, see Table 1.

### Results

### Figure 4

DNA oligonucleotides in peripheral blood are tested for stability..
A) Recovery and qPCR amplification of the DNA tag after cellular selection following different incubation periods and after attachment to peripheral blood lymphocytes (0, 30, and 60 min). These measures were conducted using three different DNA oligonucleotide tags (Code-1OS-1-Oligo-1, 2 or 3, Table 1). Ct= threshold cycle for PCR amplification.

### Example 2 (E2): Two tagged polystyrene beads comprising one type of carrier, one type of cargo and either of two types of surface molecules

### Summary of example

This is an example where the sample was PBMCs.

The carrier and cargo was in the form of a commercially available fluorescein containing polystyrene bead (Spherotech, #SVFP-0552-5, USA, Illinois). The polystyrene beads are surface modified with streptavidin to be receptive for binding of biotinylated molecules. These streptavidin coated polystyrene beads were surface coated with either anti-CD8 or anti-CD19 antibodies, as well as biotinylated DNA-oligonucleotide tags to respectively encode the type of surface molecule i.e the antibody specificity.

The tagged molecules were contacted with PBMCs where after CD8 positive and CD19 positive cells were identified by staining with fluorochrome labeled anti-CD8 and anti-CD19 antibodies. In addition, the polystyrene beads contain fluorescein, and specific delivery to either CD8 positive or CD19 positive cells was immediately identified by flow cytometry. CD8 positive and CD19 positive populations were separated by FACS. Separated fractions of CD8 positive cells and CD19 positive cells were analysed for associated oligonucleotide tags on cell-bound particles by qPCR analysis using tag-specific qPCR probes.

### Preparation of sample

In this example, the sample was prepared as PBMCs from peripheral blood.

### Collecting sample

As example 1.

### Modifying sample

As example 1.

### Production of tagged particles

### Synthesis

In this example, the tagged particle was a streptavidin-coated polystyrene bead (carrier) loaded with a fluorescein (Yellow particles) (cargo) which was modified with attachment of biotinylated anti-CD19 antibody or biotinylated anti-CD8 antibody (surface molecule). Finally, the particle was tagged with a biotinylated oligonucleotide (tag) to encode the specificity of the antibody.
- ***Carrier*:** As example 1.
- ***Cargo*:** As example 1.
- ***Surface molecule*:** Biotinylated anti-CD19 antibody [1G9] (ab52055) and Anti-CD8 antibody [MEM-31] (ab28090) both 1 mg/mL were from AbCam. Alternatively, other biotinylated antibodies specific for CD19 and CD8 from other sources could have been used. Each bead holds approx. 50000 binding sites for biotin. Anti-CD8 or CD19 antibodies, was added in a bead to antibody ratio of 100:1, 200:1; or 400:1. The reaction was incubated for 20 min at 4°C.
- ***Tag*:** As example 1, table 1.

### Synthesis of tagged particles

Each of the two biotinylated oligonucleotide tags were attached to the particle surface via streptavidin/biotin linkage. Each particle was tagged with 2 to 10 oligonucleotides, prior to surface molecule addition, in such a way that anti-CD19-streptavidin is combined with Code-1OS-1-Oligo-1and anti-CD8-streptavidin is combined with Code-1OS-1-Oligo-2. The resulting oligonucleotide-tagged particles were additionally coated with anti-CD8 or anti-CD19 antibodies, in a bead to antibody ratio of 100:1, 200:1; or 400:1.

### Purification

Excess surface molecules and oligonucleotide tags were separated from particles by size-exclusion centrifugation (300kD, Pall Corporation, USA, OD300C34, or Sartorius, Germany, VS0151).

### Contacting tagged particles with sample

### Amount of sample

10-20 million PBMCs were used

### Amount of tagged particle

100 million tagged particles were used per sample.

### Conditions during contact

Cells were resuspended in 20 µl barcode-buffer (PBS/0.5% BSA/2 mM EDTA/100 µg/ml herring DNA) per staining. Tagged particles were centrifuged for 5 min, 3300 g, prior to addition to cells. After adding tagged particles, the cells were incubated 30 min, 4°C. Antibodies identifying CD8 positive and CD19 positive cell subsets, anti-CD19 (APC) and anti-CD8 (PerCP), both from BD Biosciences, were additionally added together with 0.1 µl near- IR-viability dye (Invitrogen L10119) that stains free amines. Cells were incubated 30 min, 4°C. After washing the cells twice in barcode-buffer, the cells were ready for flow cytometric acquisition. Optionally, cells were fixed in 1% paraformaldehyde O.N., 4°C, and washed twice in barcode-buffer. Fixed cells were stored for up to a week at 4°C before flow cytometric acquisition.

### Recovering tagged particles causing specific effect/change

### Apply assay for separation

Cells were sorted on a BD FACSAria equipped with three lasers (488 nm blue, 633 nm red and 405 nm violet) on the basis of fluorochrome labeled anti-CD19 (APC) and anti-CD8 (PerCP) antibodies.

### Wash

Before separation of cells the sample was washed in 400 µL barcode-buffer (PBS/0.5% BSA/2 mM EDTA/100 µg/ml herring DNA) followed by centrifugation 5 min, 490 g, with subsequent removal of supernatant and resuspension in 200 µL barcode-buffer.

### Separate tagged particles

Cells labeled with fluorochrome labeled anti-CD19 or anti-CD8 antibodies, respectively, were sorted into tubes that had been pre-saturated overnight in 2% BSA and contain 200 µl barcode-buffer to increase the stability of the oligonucleotide tag on particles that follow with the sorted cells. The sorted cells were centrifuged 5 min, 5000 g, to allow removal of all excess buffer and to remove tagged particles not bound to cells.

### Identifying particle components by the unique particle tag

Secure tags on separated particles
Sorted cells are stored at -80°C.

### Apply method for deconvolution of tag information

The abundance of oligonucleotide tags, Code-1OS-1-Oligo-1 (anti CD19) and Code-1OS-1-Oligo-2 (anti-CD8) was determined in the individual sorted fractions by quantitative PCR using the forward primer Code-1OS-F1 and the reverse primer Code-1OS-R1 together with the Code-1OS-1-Oligo-1-specific and Code-1OS-1-Oligo-2-specific Q-PCR probes Code-QProbe-1, and Code-QProbe-2. (see Table 1, example 1) .

### Results

### Figure 5

Specific delivery of polystyrene beads to CD8 positive lymphocytes are accomplished following surface attachment of anti-CD8 antibodies and DNA oligonucleotides. The interaction can be tracked through flow cytometry following mixture of peripheral blood lymphocytes and polystyrene beads coated with anti-CD8 antibodies and DNA oligonucleotides tags.
A) The figure shows specific interaction with CD8 T cells (PerCp channel) and the fluorescent dye (cargo) in the polystyrene beads (AmCyan). In contrast, only minimal attachment of polystyrene beads to CD8 T cell subsets in peripheral blood lymphocytes were observed when these bead did not contain anti-CD8 antibodies.
B) The figure shows mixture of coated and non-coated polystyrene with DNA oligonucleotide tags. For flow cytometry, cells were stained with anti-CD8 PerCp after the attachment of polystyrene beads. Polystyrene beads are visible in the AmCyan channel.

### Figure 6

DNA oligonucleotide tags on polystyrene beads can be used to track particles associated with a given cell type. A) CD8 positive cells were separated from the remaining cells (CD8 negative cells).qPCR analysis of oligonucleotide tags associated with either fraction revealed that the oligo tag (Code-1OS-1-Oligo-2) encoding the carrier modified with anti CD8 antibody as surface molecule was only detected in the CD8 positive cell separation where as the CD8 negative cell separation contained no detectable amount of Code-1OS-1-Oligo-2. This was found for all three tested ratios of antibody: particle. Ct= threshold cycle for PCR amplification

### Example 3 (E3): Tagged liposomes comprising one type of carrier, one type of cargo and two types of surface molecules.

### Example summary

This is an example where the sample is PBMCs. The carrier and cargo is ready formed as a commercially available doxorubicin-loaded liposome. The surface molecules are biotinylated anti-CD19 and biotinylated anti-CD8 antibodies, respectively, bound to lipidated streptavidin in a 1:1 ratio. The tag is a biotin-modified oligonucleotide, which is also immobilised onto the lipidated streptavidin-antibody complex. The lipidated streptavidin-antibody-tag complex is finally grafted into the liposome carrier. The tagged molecule is contacted with PBMCs where after CD8 positive and CD19 positive cells are identified by staining with fluorochrome labeled anti-CD8 and anti-CD19 antibodies and separated by FACS. Separated fractions of CD8 positive cells and CD19 positive cells are analysed for associated oligonucleotide tags on cell-bound particles by qPCR analysis using tag-specific qPCR probes.

### Preparation of sample

### Collecting sample

Blood is obtained from the Danish blood bank (Dept. clinical immunology, 'Rigshospitalet', Denmark).

### Modifying sample

PBMCs are isolated from whole blood by density gradient centrifugation. The density gradient medium, Lymphoprep (Axis-Shield), which consists of carbohydrate polymers and a dense iodine compound, facilitate separation of the individual constituents of blood. Blood samples are diluted 1:1 in RPMI (RPMI 1640, GlutaMAX, 25mM Hepes; gibco-Life technologies) and carefully layered onto the Lymphoprep. After centrifugation, 30 min, 390 g, PBMCs together with platelets are harvested from the middle layer of cells. The isolated cells, the buffy coat (BC), is washed twice in RPMI and cryopreserved at -150°C in fetal calf serum (FCS; gibco-Life technologies) containing 10% dimethyl sulfoxide (DMSO; Sigma-Aldrich).

Prior to use BCs are thawed in 10 ml, 37°C, RPMI with 10% fetal bovine serum (FBS), centrifuged 5 min, 1500 g, and washed in 10 ml RPMI with 10% FBS. All washing of cells refer to centrifugation 5 min, 490 g, with subsequent removal of supernatant. 10-20 million cells per sample are washed in 400 µl barcode-buffer (PBS/0.5% BSA/2 mM EDTA/100 µg/ml herring DNA) and resuspended in this buffer to approximately 20 µl per sample.

### Production of tagged particles

### Synthesis

In this example, the tagged particle is a liposome (carrier) loaded with doxorubicin (cargo), which is modified with lipidated streptavidin conjugated with either biotinylated anti-CD19 antibody or biotinylated anti-CD8 antibody (surface molecule). Finally, the lipidated streptavidin antibody complex is further complexed with a biotinylated oligonucleotide (tag) to encode the specificity of the antibody and the entire streptavidin-antibody-tag complex is grafted into the particle.
- ***Carrier*:** Pegylated liposomes (carrier) ready loaded with doxorubicin (cargo) is purchased from Avanti Polar Lipids (Dox-NP® # 300102 concentration ∼1,5E11 liposomes/mL = 0,25 nM, info from supplier) or a similar pre-loaded carrier is purchased from another commercial source. The carriers are approximately 100 nm.
- ***Cargo*:** Doxorubicin is ready pre-loaded into carrier by supplier.
- ***Surface molecule*:** Biotinylated anti-CD19 antibody [1G9] (ab52055) and anti-CD8 antibody [MEM-31] (ab28090) both 1 mg/mL are from AbCam. Alternatively, other biotinylated antibodies specific for CD19 and CD8 from other sources can be used. Streptavidin (# S4762 Sigma-Aldrich or similar) is lipidated by reacting with DSPE-PEG-NHS, MW 3400, (#PG2-DSNS-3k from Nanaocs) in a molar ratio of approximately 1:2. Briefly, streptavidin is dissolved to 1 mg/mL in PBS pH 7,2. DSPE-PEG-NHS is dissolve to 1 mM in H₂O. 20 µL, 1 mM DSPE-PEG-NHS is added to 1 mL, 1 mg/mL streptavidin in PBS, pH 7,2. The reaction is incubated for 1 h at 30 degrees to allow lipidation-reaction on streptavidin. Biotnylated antibodies are subsequently bound to lipidated streptavidin by mixing in a 1:1 molar ratio (1 µM each) in PBS, pH 7,2 and incubating 30 min at r.t. No further purification of surface molecules are performed.
- ***Tag*:** Two oligonucleotide tags both containing 5' biotin, Code-1OS-1-Oligo-1 and Code-1OS-1-Oligo-2, as described in table 1 are purchased from DNA-Technology.

### Synthesis of tagged particles

Each of the two biotinylated oligonucleotide tags are combined in a 1:1 molar ratio with the antibody-streptavidin complexes (1 µM each in PBS, pH 7,2.) in such a way that anti-CD19-streptavidin is combined with Code-1OS-1-Oligo-1 (anti CD19) and anti-CD8-streptavidin is combined with and Code-1OS-1-Oligo-2 (anti-CD8). The two reactions are incubated for 1 h at 30 degrees to allow complex formation. The resulting lipidated streptavidin-antibody-tag complexes are grafted onto doxorubicin loaded liposomes in two reaction by mixing the two respective complex variations with liposomes in a molar ratio of 1:10000 (1nM streptavidin-antibody-tag complexes to 10 uM lipid constituent of liposome) and incubated in a heating block (Thermomixer comfort) for 1 hour at 60 °C during continuous rotation where after the suspension is cooled down. The number of lipids in a 100 nm size liposome is about 80000. Thus, the number of streptavidin-antibody-tag complexes per liposome is in the range of 8.

### Purification

Free doxorubicin, excess surface molecules and oligonucleotide tags are separated from particles by gel filtration chromatography using a 4B sepharose column (Sigma-Aldrich).

### Contacting tagged particles with sample

### Amount of sample

10-20 million PBMCs are used

### Amount of tagged particle

Approximately 10 million tagged particles of both species (CD19 targeted and CD8 targeted) are used.

### Conditions during contact

Cells are resuspended in 20 µl barcode-buffer (PBS/0.5% BSA/2 mM EDTA/100 µg/ml herring DNA) per staining. Tagged particles are centrifuged for 5 min, 3300 g, prior to addition to cells. After adding tagged particles, the cells are incubated 30 min, 37°C. Anti-CD19 (APC) and anti-CD8 (PerCP), both from BD Biosciences, are additionally added. Cells are incubated 30 min, 4°C. After washing the cells twice in barcode-buffer, the cells are ready for flow cytometric acquisition. Optionally, cells are fixed in 1% paraformaldehyde O.N., 4°C, and washed twice in barcode-buffer. Fixed cells are stored for up to a week at 4°C

### Recovering tagged particles causing specific effect/change

### Apply assay for separation

Cells are sorted on a BD FACSAria equipped with three lasers (488 nm blue, 633 nm red and 405 nm violet) on the basis of their labeling with fluorochrome labeled anti-CD19 and anti-CD8 antibodies.

### Wash

Before separation of cells, the sample is washed in 400 µL barcode-buffer (PBS/0.5% BSA/2 mM EDTA/100 µg/ml herring DNA) followed by centrifugation 5 min, 490 g, with subsequent removal of supernatant and resuspension in 200 µL barcode-buffer.

### Separate tagged particles

Cells labeled with fluorochrome labeled anti-CD19 or anti-CD8 antibodies, respectively, are sorted into tubes that have been pre-saturated overnight in 2% BSA and contain 200 µl barcode-buffer to increase the stability of the oligonucleotide tag on particles that follow with the sorted cells. The sorted cells are centrifuged 5 min, 5000 g, to allow removal of all excess buffer and to remove tagged particles not bound to cells

### Identifying particle components by the unique particle tag

Secure tags on separated particles
Sorted cells are stored at -80 °C

### Apply method for deconvolution of tag information

The abundance of oligonucleotide tags, Code-1OS-1-Oligo-1 (anti CD19) and Code-1OS-1-Oligo-2 (anti-CD8) was determined in the individual sorted fractions by quantitative PCR using the forward primer Code-1OS-F1 and the reverse primer Code-1OS-R1 together with the Code-1OS-1-Oligo-1 and Code-1OS-1-Oligo-2 specific Q-PCR probes Code-QProbe-1OS-1-1, and Code-QProbe-1OS-1-2. For sequences, see Table 1, example 1.

### Example 4 (E4): Constructing a tagged particle with a unique tag describing each element

### Example summary

This is an example where a simple carrier in the form of a liposome is formed by conventional methods as performed by a person skilled in the art. The liposome is grafted with trace amounts of a 3' lipid-modified (liposome anchor) and 5' phosphate modified start oligonucleotide, which forms the basis for building an oligonucleotide tag, which will encode the nature of the particle (see tag design table 3 and figure 8). The first coding element, Codon A (5' phosphate modified oligo) together with an Ax oligo which has a complimentary region with both start oligo and Codon A, is combined with start oligo and Codon A is enzymatically ligated onto the start oligo and will form a first code to identify the nature of the liposome carrier. After forming the liposome, doxorubicin is loaded into the liposome by remote loading by means of a salt gradient. Codon B (5' phosphate modified) together with a Bx oligo the B codon is is combined with start oligo+Codon A and ligated onto Codon A to encode for doxorubicinby adding the Codon B tag (second code) through extension of Codon A. Finally, a targeting antibody is lipidated and grafted into the liposome. Codon C (5' phosphate modified) is included together with a Cx oligo and Codon C is ligated onto Codon B. Codon C will form a tag for the identity of targeting antibody. Finally after mixing particles a Terminator-oligo T together with a Tx oligo is ligated onto the C codon.

### Preparation of CaAcO₂ loaded liposomes for remote-loading of cargo

1. Mix the appropriate volume of hydrogenated soybean phosphatidylcholine (HSPC), PEG2000-PE and cholesterol (molar ratio of 55:5:40 all lipids from Avanti Polar Lipids) in accordance with table 2 in a 2 ml eppendorf tube.
2. Evaporate solvent using a steady stream of argon.
3. To remove all traces of chloroform freeze dry the lipid film under vacuum for approx. 2h
4. Rehydrate the lipid film in 100 µl EtOH by placing the tube in a thermomixer at 65°C and 1400 rpm for 10 min.
5. Form multilamellar vesicles (MLV's) by adding 900 µl of a 200 mM CaAcO₂ solution and incubate in thermomixer (65°C and 1400 rpm) for 1h
6. Assemble the extruder (Avanti Polar Lipids) according to the manual using filters with pore size of 100 nm and extrude the liposome solution 25 times.

**Table 2**

| **Lipid name** | **Mw** | **Molar ratio** | **Stock (mg/ml)** | **µmole** | **weight mg** | **vol (µl)** |
|---|---|---|---|---|---|---|
| mPEG2000-PE | 2805,54 | 5,0 | 20 | 5,00 | 14,03 | 701,41 |
| HSPC | 762,1 | 55,0 | 100 | 55,00 | 41,92 | 419,21 |
| Cholesterol | 386 | 40,0 | 100 | 40,00 | 15,44 | 154,41 |
| | | | | | | |
| **Total volume** | 1 ml | | | | | |
| **Lipid conc** | 100,00 mM | | | | | |

### Adding tag to liposome

1. A start oligonucleotide, (fig 8) (TAGCTCTGTACGTCTATGCGAAAGTZ Z = 3' DSPE-PEG lipidated anchor oligonucleotide tag (SEQ ID NO: 84), synthesized by DNA Technology, Denmark) is mixed with an Ax1 oligo, and a codon oligo (5' phosphate modified), Codon A1 (encoding the nature of the liposome), in a 1:2:3 ratio (5 µM:10 µM:20 µM) in 100 µL H₂O. For A oligo and Ax oligo sequences see table 3.
2. The reaction is heated to 80°C and allowed to cool to room temperature.
3. The annealed oligonucleotides are mixed with T4 DNA Ligase (NEB #M0202S) and reaction buffer (50 mM Tris-HCI, 10 mM MgCl₂, 1 mM ATP, 1 mM DTT, pH 7.5) and incubated at 16°C according to manufacturer's protocol.
4. The resulting ligated Start oligo and Codon A (Tag) is diluted in H₂O and added to the liposome solution in a molar ratio of 1:10000 anchor: lipid (5:55:40:0,01 PEG2000-PE:HSPC:Cholesterol:Tag in mM). The number of lipids in a 100 nm size liposome is about 80000. Thus, the number of tags per liposome is in the range of 8.
5. The solution is incubated in a thermomixer (65°C and 1400 rpm) for 1h.
6. Following, liposomes are dialyzed against 0,9% NaCl O/N at 4°C

### Loading of cargo

1. Doxorubicin hydrochloride (#D1515 SIGMA) is dissolved to 5 mg/mL in H₂O
2. Mix Ca2⁺ loaded liposomes with dissolved doxorubicin in 100 mM citrate pH 6,0 in a 1:1 molar ratio (lipid:doxorubicin, 10mM:10mM).
3. Incubate in thermomixer at 65°C and 300 rpm for 20 min.
4. Purify using Zeba spin columns equilibrated in PBS pH 7.4.

### Adding tag to liposome

1. Oligo Bx1 (5 µM) and Codon B1 (10 µM) (encoding the nature of cargo) are mixed with liposome solution. 30 min 30°C in PBS pH 7.4.
2. The annealed oligonucleotides are mixed with T4 DNA Ligase (NEB #M0202S) and reaction buffer (50 mM Tris-HCl, 10 mM MgCl₂, 1 mM ATP, 1 mM DTT, pH 7.5) and incubated at 16°C according to manufacturer's protocol for ligation of Codon A1 with Codon B1 as directed their ennealing to oligo Bx1 (see figure 8).
3. The solution is incubated in thermomixer (65°C, 1400 rpm, 10 min) for heat inactivation of DNA ligase.
4. Purify using Zeba spin columns equilibrated in PBS pH 7.4

### Post-insertion of lipidated targeting antibody

1. For post-insertion of lipidated IgG onto preformed liposomes mix appropriate amount of liposomes and ice-cold PBS pH 7.4.
2. Anti-CD19 antibody [2E2B6B10] (ab31947. AbCam) is lipidated by reacting with DSPE-PEG-NHS, MW 3400, (#PG2-DSNS-3k from Nanaocs) in a molar ratio of 1:2. Briefly, DSPE-PEG-NHS is dissolved to 1 mM i H₂O. 20 µL, 1 mM DSPE-PEG-NHS is added to 1 mL, 1 mg/mL anti-CD19 antibody in PBS, pH 7,2. The reaction is incubated for 1 h at 30 degrees to allow lipidation-reaction on anti-CD19 antibody.
3. Subsequently, lipidated anti-CD19 antibody is mixed with liposome solution in a ratio of 1:2500 Ab:lipid and the solution is transferred to a dialysis tube (MWCO 14.000) and dialysed against 1 xPBS pH 7.4 O/N. The number of lipids in a 100 nm size liposome is about 80000. Thus, the number of Ab's per liposome is in the range of 32.
4. Change dialysis buffer and continue dialysis for 24-48h.

### Adding tag to liposome

1. Oligo Cx1 (5 µM) and Codon C1 (10 µM) (encoding the nature of surface molecule, i.e. targeting antibody) are mixed with liposome solution. 30 min 30°C in PBS pH 7.4.
2. The annealed oligonucleotides are mixed with T4 DNA Ligase (NEB #M0202S) and reaction buffer (50 mM Tris-HCl, 10 mM MgCl2, 1 mM ATP, 1 mM DTT, pH 7.5) and incubated at 16°C according to manufacturer's protocol for ligation of Codon B1 with Codon C1 as directed by their annealing to oligo Cx1 (see figure 8).
3. The solution is incubated in thermomixer (65°C, 1400 rpm, 10 min) for heat inactivation of DNA ligase.
4. Purify using Zeba spin columns equilibrated in PBS pH 7.4

### Example 5 (E5): Synthesis of a plurality of tagged particles (liposomes) with surface molecules and cargo - using a mix and split approach.

### Example summary

This is an example of a 1000 member library of tagged particles, particle with a unique composition and with a unique tag, that is continuously build through ligation of DNA nucleotide sequences, which identify each individual component of the particle. The 1000 different particles are generated through 'mix and split' synthesis of 10 different liposome carriers, 10 different cancer-drug cargos and 10 different antibody surface molecules, resulting in 10x10x10=1000 different particles, each carrying an individual tag comprising three sub-segments. Sub-segment A (Codon oligo A) is coding for the identity of the carrier, sub-segment B (Codon oligo B) is coding for the identity of the cargo, and sub-segment C (Codon oligo C) is coding for the identity of the surface molecule (Table 3)

**Table 3:**

| Lowercase letters in sequences in the table below denote 5' phosphate labeled nucleotides. Z denotes an anchor, e.g. 3' DSPE-PEG-NHS, MW 2 - 3.4 kDa | | | | | |
|---|---|---|---|---|---|
| Tag oligo | | Tag annealing oligo | | Encoded component | |
| ID | Tag Sequence 3'-5' | ID | Tag annealing sequence 5'-3' | Type | Description |
| S | | - | - | - | - |
| A1 | | Ax1 | | Carrier | HSPC : mPEG2000-PE: Cholesterol |
| A2 | | Ax2 | | Carrier | HSPC : mPEG2000-PE: Cholesterol |
| A3 | | Ax3 | | Carrier | HSPC : mPEG2000-PE: Cholesterol |
| A4 | | Ax4 | | Carrier | HSPC : mPEG2000-PE: Cholesterol |
| A5 | | Ax5 | | Carrier | HSPC : mPEG2000-PE: Cholesterol |
| A6 | | Ax6 | | Carrier | HSPC : mPEG5000-PE : Cholesterol |
| A7 | | Ax7 | | Carrier | HSPC : mPEG5000-PE : Cholesterol |
| A8 | | Ax8 | | Carrier | HSPC : mPEG5000-PE : Cholesterol |
| A9 | | Ax9 | | Carrier | HSPC : mPEG5000-PE : Cholesterol |
| A1 0 | | Ax10 | | Carrier | HSPC : mPEG5000-PE : Cholesterol |
| B1 | | Bx1 | | Cargo | doxorubicin |
| B2 | | Bx2 | | Cargo | epirubicin |
| B3 | | Bx3 | | Cargo | idarubicin |
| B4 | | Bx4 | | Cargo | cyclophosp hamide |
| B5 | | Bx5 | | Cargo | paclitaxel |
| B6 | | Bx6 | | Cargo | docetaxel |
| B7 | | Bx7 | | Cargo | etoposide |
| B8 | | Bx8 | | Cargo | vincristine |
| B9 | | Bx9 | | Cargo | mechlorat hmine |
| B10 | | Bx10 | | Cargo | temozolom ide |
| C1 | | Cx1 | | Surface molecule | Anti-CD 19 [2E2B6B10] ab31947 |
| | | | | | |
| C2 | | Cx2 | | Surface molecule | Anti-CD 19 [EPR5906] (ab134114) |
| C3 | | Cx3 | | Surface molecule | Anti-CD19 [EPR2230(N)] (ab197895) |
| C4 | | Cx4 | | Surface molecule | Anti-CD 19 [3G7] (ab140981) |
| C5 | | Cx5 | | Surface molecule | Anti-CD20 [L26] (ab9475) |
| C6 | | Cx6 | | Surface molecule | Anti-CD20 [EP459Y] (ab78237) |
| C7 | | Cx7 | | Surface molecule | Anti-CD20 [MEM-97] (ab8237) |
| C8 | | Cx8 | | Surface molecule | Anti-CD22 [2H1C4] (ab181771) |
| C9 | | Cx9 | | Surface molecule | Anti-CD22 [EP498Y] (ab33859) |
| C10 | | Cx10 | | Surface molecule | Anti-CD22 [RFB-4] (ab112182) |
| T | | Tx1 | | - | - |

### Preparation of CaAcO₂ loaded liposomes for remote-loading of cargo

1. Volumes of hydrogenated soybean phosphatidylcholine (HSPC), mPEG2000-PE/mPEG5000-PE and cholesterol in 10 different molar ratios are mixed in accordance with table 3 in 10x2 ml eppendorf tubes.
2. Evaporate solvent from each tube using a steady stream of argon.
3. To remove all traces of chloroform freeze dry the lipid films under vacuum for approx. 2h
4. Rehydrate the lipid films in 100 µl EtOH by placing the tubes in a thermomixer at 65°C and 1400 rpm for 10 min.
5. Form multilamellar vesicles (MLV's) by adding 900 µl of a 200 mM CaAcO₂ solution to each tube and incubate in therm-mixer (65°C and 1400 rpm) for 1h
6. Assemble the extruder (Avanti Polar Lipids) according to the manual using filters with pore size of 100 nm and extrude the 10 liposome solutions 25 times.

### Adding tag to liposomes

1. 10 tubes each containing Start oligo (Table 3 and Figure 8) is mixed with one of oligo Ax1 to oligo Ax10 and one of Codon oligo A1 to A10, respectively, encoding the nature of the 10 different liposomes. Start oligo, Ax oligo and Codon oligo Ax is mixed in a 1:2:3 ratio (5 µM:10 µM:20 µM) in 100 µL H₂O.
2. The 10 reactions are heated to 80°C and allowed to cool to room temperature.
3. Each of the 10 annealed oligonucleotide reactions are mixed with T4 DNA Ligase (NEB #M0202S) and reaction buffer (50 mM Tris-HCl, 10 mM MgCl₂, 1 mM ATP, 1 mM DTT, pH 7.5) and incubated at 16°C according to manufacturer's protocol.
4. The solutions are incubated in thermomixer (65°C, 1400 rpm, 10 min) for heat inactivation of DNA ligase.
5. The resulting 10 ligated Start oligos and Codon Ax oligos are diluted in H₂O and added to the 10 liposome forl'mulations in a molar ratio of 1:10000 Tag:lipid (0,010:100 mM). The number of lipids in a 100 nm size liposome is about 80000. Thus, the number of tags per liposome is in the range of 8.
6. The solutions are incubated in a thermomixer (65°C and 1400 rpm) for 1h.

### Mix liposomes

1. The 10 different tagged liposome formulations are mixed
2. Following, the liposome mix is dialyzed against 0,9% NaCl O/N at 4°C

### Split liposomes

1. 10 equal volumes of liposome mix is aliquoted into tubes

### Loading of cargo

1. 10 small-molecule cancer drugs (cargo), doxorubicin, epirubicin, idarubicin, cyclophosphamide, paclitaxel, docetaxel, etoposide, vincristine, mechlorathmine, temozolomide and daunorobicin (SIGMA, Cayman, AbCam and Enzo Life Sciences) are dissolved individually to 5 mg/mL in H2O.
2. Into each of the 10 Ca2⁺ loaded liposome aliquots mix one of the dissolved cargo drugs and 100 mM citrate pH 6,0 according to table 3.
3. Incubate each of the 10 tubes in thermomixer at 65°C and 300 rpm for 20 min.
4. Purify drug loaded liposomes using Zeba spin columns equilibrated in PBS pH 7.4

### Adding tag to liposome

1. Oligo Bx1-10 (5 µM) and Codon B1-10 (10 µM) (encoding the nature of cargos respectively) are mixed with the 10 liposome solutions respectively. 30 min 30°C in PBS pH 7.4.
2. The 10 reactions of annealed oligonucleotides are mixed with T4 DNA Ligase (NEB #M0202S) and reaction buffer (50 mM Tris-HCl, 10 mM MgCl2, 1 mM ATP, 1 mM DTT, pH 7.5) and incubated at 16°C according to manufacturer's protocol for ligation of Codon A1-10 with Codon B1-10 respectively as directed by their annealing to oligo Bx1-10 respectively (see figure 8).
3. The solutions are incubated in thermomixer (65°C, 1400 rpm, 10 min) for heat inactivation of DNA ligase.

### Mix liposomes

1. The 10 tubes containing 10 different liposome formulations each loaded with 10 different cargo drugs are mixed.
2. The combined liposomes are purified using Zeba spin columns equilibrated in PBS pH 7.4

### Split liposomes

1. 10 equal volumes of mixed liposomes with drugs loaded are aliquoted into tubes.

### Post-insertion of lipidated targeting antibody

1. For post-insertion of lipidated IgG onto preformed liposomes mix liposomes and ice-cold PBS pH 7.4.
2. 10 antibodies, four anti-CD19 antibodies ([2E2B6B10] ab31947, [EPR5906] (ab134114), [EPR2230(N)] (ab197895) and [3G7] (ab140981), three anti-CD20 antibodies ([L26] (ab9475), [EP459Y] (ab78237) and [MEM-97] (ab8237) and three anti-CD22 antibodies ([2H1C4] (ab181771), [EP498Y] (ab33859) and [RFB-4] (ab112182)) all from AbCam are lipidated by reacting with DSPE-PEG-NHS, MW 3400, (#PG2-DSNS-3k from Nanaocs) in a molar ratio of 1:2. Briefly, DSPE-PEG-NHS is dissolve to 1 mM in H₂O. 20 µL, 1 mM DSPE-PEG-NHS is added to 1 mL, 1 mg/mL antibody in PBS, pH 7,2. The reaction is incubated for 1 h at 30 degrees to allow lipidation-reaction on antibody.
3. Subsequently the 10 lipidated antibodies are mixed respectively with a liposome solution in a 1:2500 Ab:lipid ratio and the solution is transferred to a dialysis tube (MWCO 14.000) and dialysed against 1 xPBS pH 7.4 O/N. The number of lipids in a 100 nm size liposome is about 80000. Thus, the number of Ab's per liposome is in the range of 32.
4. Change dialysis buffer and continue dialysis for 24h.

### Adding tag to liposome

1. Oligo Cx1-10 (5 µM) and Codon C1-10 (10 µM) (encoding the nature of surface molecules respectively) are mixed with the 10 liposome solutions respectively. 30 min 30°C in PBS pH 7.4.
2. The 10 reactions of annealed oligonucleotides are mixed with T4 DNA Ligase (NEB #M0202S) and reaction buffer (50 mM Tris-HCl, 10 mM MgCl2, 1 mM ATP, 1 mM DTT, pH 7.5) and incubated at 16°C according to manufacturer's protocol for ligation of Codon B1-10 with Codon C1-10 respectively as directed by their annealing to oligo Cx1-10 respectively (see figure 8).
3. The solution is incubated in thermomixer (65°C, 1400 rpm, 10 min) for heat inactivation of DNA ligase.

### Mix liposomes

1. The 10 tubes containing combinations of 10 different liposome formulations each loaded with 10 different cargo drugs and modified with 10 different surface molecules are mixed.
2. The combined liposomes are purified using Zeba spin columns equilibrated in PBS pH 7.4.
3. The library of tagged particles is now ready for contacting with a sample.

### Example 6 (E6): Contacting a library of tagged particles with a sample.

### Example summary

In this example, the sample is anticoagulated, EDTA treated whole blood. A library of tagged particles (as described in example 5) consisting of a combination of different liposome formulations, different anti-cancer drugs as cargo and different anti-CD19, anti-CD20 and anti-CD22 antibodies as surface molecule formulations and the tagged particles are contacted with anticoagulated whole blood for 0-24 hours. At 0 hours, 0,5 hours, 2 hours, 8 hours and 24 hours apoptotic cells are identified by staining with fluorochrome labeled annexin V and separated by FACS. Separated fractions of apoptotic cells are analysed for associated oligonucleotide tags on cell-bound particles by Next Generation deep sequencing.

### Preparation of sample

In this example, the sample is prepared as anticoagulated, EDTA treated whole blood.

### Collecting sample

Blood is obtained from the Danish blood bank (Dept. clinical immunology, 'Rigshopitalet', Denmark).

### Modifying sample

Whole blood is drawn into BD Vacutainer® Plus Plastic K2EDTA Tubes.

Anticoagulated blood is further prepared by mixing 1:1 with RPMI and incubated up to 1h at 37°C before use. All subsequent washing of cells refer to washing in barcode-buffer (PBS/0.5% BSA/2 mM EDTA/100 µg/ml herring DNA) followed by centrifugation 5 min, 490 g, with subsequent removal of supernatant.

### Production of tagged particles

### Synthesis

The library of tagged liposomes with anti-cancer drugs as cargo and targeted for B cells is prepared as described in example 5.

### Contacting tagged particles with sample

### Amount of sample

1 mL sample is used

### Amount of tagged particle

1-10 million tagged particles in a volume of 50 µL is used

### Conditions during contact

Tagged particles are centrifuged for 5 min, 3300 g, prior to addition to sample. After adding tagged particles, the sample is incubated at 37°C in a humidified incubator containing 5% CO₂. At all timepoints (0 min, 30 min, 2 hours, 8 hours, and 24 hours) 200 µL sample is collected and washed twice in 400 µL barcode-buffer. Optionally, cells are fixed in 1% paraformaldehyde O.N., 4°C, and washed twice in barcode-buffer. Fixed cells are stored for up to a week at 4°C

### Recovering tagged particles causing specific effect/change

### Apply assay for separation

Cells are sorted on a BD FACSAria equipped with three lasers (488 nm blue, 633nm red and 405 nm violet) on the basis of externalized phosphatidylserine which is one of the leading indicators of apoptosis. Externalized phosphatidylserine is detected by labeling cells with Annexin V, Alexa Fluor® 488 Conjugate from Life Technologies.

### Wash

Before separation of cells, the sample is washed in 400 µL barcode-buffer (PBS/0.5% BSA/2 mM EDTA/100 µg/ml herring DNA) followed by centrifugation 5 min, 490 g, with subsequent removal of supernatant and resuspension in 200 µL barcode-buffer.

### Separate tagged particles

Cells labeled with Annexin V, Alexa Fluor® 488 Conjugate are sorted into tubes that have been pre-saturated overnight in 2% BSA and contain 200 µl barcode-buffer to increase the stability of the oligonucleotide tag on particles that follow with the sorted cells. The sorted cells are centrifuged 5 min, 5000 g, to allow removal of all excess buffer and to remove tagged particles not bound to cells.

### Identifying particle components by the unique particle tag

Secure tags on separated particles
Sorted cells are stored at -80 °C.

PCR amplify oligonucleotide tags on tagged particles associated with sorted cells. Forward and reverse primers complimentary to the Start oligo and the Terminator oligo of the tag are used for standard PCR. Both forward and reverse primers also contain adaptor sequences for making the PCR products compatible with Next Generation sequencing. The forward primer is adapted with an A key sequence and the reverse primer is adapted with a P1 key sequence for Ion Torrent sequencing. All PCR reactions and PCR products are performed and prepared as recommended by Ion Torrent, Life Technologies.

### Apply method for deconvolution of tag information

The abundance of individual oligonucleotide tags in the separated fraction of apoptotic cells is determined and compared to sorted fractions of non-apoptotic cells by deep sequencing, Ion Torrent, Life Technologies, using commercial service providers.

The sequence analysis is performed using public analysis tool developed by CBS, DTU.

### Results:

Abundant oligonucleotide tags in fractions of apoptotic cells will indicate the composition and nature of stable and potent particles with ability to kill cells. Such particles have high therapeutic value.

### Example 7 (E7): Testing for targeted delivery to cancer tissue in vivo.

### Example summary

In the following example, we are testing for *in vivo* delivery of a library of particles to a tumor lesion. The model used is a xenograft model of a human tumor, Merkel Cell Carcinoma. This cancer type is induced by an oncogenic virus, Merkel Cell Polyomavirus and can potentially be eliminated by virus-specific T cells (as previously described, Lyngaa et al Clin Can Res, 2014). Here, we identify particle formulations that can specifically deliver cytokines (IL2) and immune-checkpoint blockade (anti-PD1) to the tumor lesion as a means to enhance the therapeutic efficacy of adoptively transferred virus-specific T-cells. Particles leading to enhanced T-cell mediated tumor destruction will be selected for further optimization.

### Preparation of sample

### Collecting sample

Take tumor line in culture (alternatively, use thawed tumor digest. This does not need culturing). Split cells 1:1 the day before injection. Harvest cells and dissolve 1E6 cells in 100 µl injection solution (i.e. 50 µl of Matrigel + 50 µl of RPMI). Inject 1E6 cells s.c. in the flank of NOG mice. Monitor tumor growth once or twice per week for the following three weeks.

### Modifying sample

Animals are sacrificed when tumors exceed 15 mm in any dimension or when the average of two dimensions is higher than 12 mm. Tumors are collected. Tumor fragments are snap-frozen for DNA extraction.

### Production of tagged particles

### Synthesis

- ***Carrier*:** As in example 3.
- ***Cargo*:** Cytokine, IL2 (Biolegend) or humanized anti-PD1 monoclonal antibody. Different ratios/different concentrations.
- ***Surface molecule*:** Mouse monoclonal antibodies against fibronectin or the alternatively-spliced A1 domain of tenascin-C, specifically upregulated in the tumor microenvironment
- **Tag:** As described in example 4-6.

### Synthesis of tagged particles

As in example 5.

### Purification

As in examples 5.

### Contacting tagged particles with sample

### Amount of sample

3-4 mice are injected per group

### Amount of tagged particle

A library of 1000 different particle will be injected into each mouse. The number of particles for each individual formulation will range from 100-10.000. Optimal amount will be titrated.

### Conditions during contact

The liposome library will be injected *i.v.* in the tail vein of the mice, 6 hrs prior to injection of tumor-specific T-cells using the same route. Particles will distribute in the organism under normal physiological conditions.

### Recovering tagged particles causing specific effect/change

Apply assay for separation

When mice are sacrificed, both tumor tissue and healthy tissue will be resected. Fragments from each tissues will be collected to assess the particle association.

### Wash

Not applied.

### Separate tagged particles

Not applied.

### Identifying particle components by the unique particle tag

Secure tags on separated particles
DNA is extracted from both healthy tissue and tumor tissue, oligonucleotide tag sequences are amplified using sample identification-specific forward primers. Performed as described in example 6.

### Apply method for deconvolution of tag information

Amplified DNA oligonucleotides from both healthy and tumor tissues is sequenced to identify particle characteristics associated with tumor specific delivery and enhanced tumor rejection capabilities of adoptively transferred T-cells. Performed as described in example 6.

### Example 8 (E8): Synthesis and screening of a library of four tagged polystyrene nanoparticles (PN's) comprising one type of carrier, one type of cargo and one of four types of surface molecules.

### Example summary

The synthesis of tagged particles is visualized in figure 10. The results are shown in figure 11. This is an example where the sample was PBMCs.

The cargo and was carrier in the form of commercially available AmCyan containing polystyrene nanoparticles (Spherotech, #SVFP-0552-5, USA, Illinois). The polystyrene particles are surface modified with streptavidin to be receptive for binding of biotinylated molecules. These streptavidin coated polystyrene particles were further surface coated either with biotinylated anti-CD4, biotinylated anti-CD8, biotinylated anti-CD19 antibodies or no antibodies, as well as with biotinylated DNA-oligonucleotide tags to respectively encode the type of surface molecule i.e the antibody specificity on each of the four types of particles.

The tagged molecules were contacted with PBMCs where after CD4 positive, CD8 positive and CD19 positive cells were identified by staining with fluorochrome labeled anti CD4, anti-CD8 and anti-CD19 antibodies. In addition, the PN's contain AmCyan, and their specific delivery to either CD4 positive, CD8 positive or CD19 positive cells was detected as AmCyan staining. CD4 positive, CD8 positive and CD19 positive populations were separated and collected by FACS. Separated fractions of cells were analyzed for oligonucleotide tags associated with cell-bound particles by SYBR green qPCR analysis using tag-specific PCR primers.

### Preparation of sample

In this example, the sample was prepared as PBMCs from peripheral blood.

### Collecting sample

Blood was obtained from the Danish blood bank (Dept. clinical immunology, 'Rigshospitalet', Denmark).

### Modifying sample

PBMCs were isolated from whole blood by density gradient centrifugation. The density gradient medium, Lymphoprep (Axis-Shield), which consists of carbohydrate polymers and a dense iodine compound, facilitate separation of the individual constituents of blood. Blood samples were diluted 1:1 in RPMI (RPMI 1640, GlutaMAX, 25mM Hepes; gibco-Life technologies) and carefully layered onto the Lymphoprep. After centrifugation, 30 min, 390 g, PBMCs together with platelets were harvested from the middle layer of cells.

### Production of tagged particles

### Synthesis

See figure 10. In this example, the tagged particle was streptavidin-coated polystyrene particles (carrier) loaded with AmCyan (cargo) which was modified with attachment of biotinylated anti-CD4 antibody, biotinylated anti-CD8 antibody or biotinylated anti-CD19 antibody (surface molecule). Particles without antibody were also prepared. Finally, the particles were tagged with a biotinylated DNA oligonucleotide (tag) to encode the specificity of the antibody. Particles absent of antibody were also encoded with specific DNA-tags.
• ***Carrier*:** Streptavidin Coated Fluorescent Yellow Particles (Spherotech, #SVFP-0552-5)
• ***Cargo*:** AmCyan as stated by the commercial supplier of the carrier.
• ***Surface molecule*:** Biotinylated anti-CD4 antibody [RPA-T4] (#300504 from BioLegend), biotinylated anti-CD8 antibody [MEM-31] (#ab28090 from AbCam) and biotinylated anti-CD19 antibody [HIB19] (#302203 from BioLegend).
• **Tag:** T1, T2, T3 and T4 as from table 4. Synthetized by DNA technology, Aarhus, Denmark.

**Table 4**

| Name | Sequence | Modification |
|---|---|---|
| F1 | AGCTCTGTACGTCTATGCGAAA (SEQ ID NO:75) | |
| T1 | | 5 = Biotin-C6 |
| T2 | | 5 = Biotin-C6 |
| T3 | | 5 = Biotin-C6 |
| T4 | | 5 = Biotin-C6 |
| R1 SYBR | CACGTCTCAGATGGGACTACG (SEQ ID NO:80) | |
| R2 SYBR | ACGTCATGCGTCTGCG (SEQ ID NO:81) | |
| R3 SYBR | CACGTCCATCCTGCGTC (SEQ ID NO:82) | |
| R4 SYBR | CACGTCTGAGTCATGGGC (SEQ ID NO:83) | |

### Synthesis of tagged particles

DNA-tagged library of polystyrene nanoparticles (PN) with AmCyan (AmC) as cargo and targeting antibodies as surface molecules.
In individual Eppendorf tubes for each of the four types, particles comprising streptavidin on their surface were coated with either biotinylated anti-CD4 antibody, anti-CD8 antibody, anti-CD19 antibody or no antibody respectively according to volumes and concentrations in Table 5 and as illustrated in figure 10. Reagents were incubated at room temperature for 30 minutes. Then DNA-tags encoding the surface molecules were linked to particles according to volumes and concentrations in Table 5. Reagents were incubated for a further 30 minutes at room temperature. The result four different types of DNA-tagged particles.

**Table 5**

| Particle name | PN (Spherote ch,#SVFP-0552-5) | biotinylated anti CD4 antibody | biotinylated anti CD8 antibody | biotinylated anti CD19 antibody | Tag | PBS buffer |
|---|---|---|---|---|---|---|
| control PN | 20 µL 1µg/µL | 0 | 0 | 0 | 2,5µL 6e10/µL T1 | 20µL |
| CD4 PN | 20 µL 1µg/µL | 20 µL 5 ng/µL αCD4 | 0 | | 2,5µL 6e10/µL T2 | 0 |
| CD8 PN | 20 µL 1µg/µL | 0 | 20 µL 5 ng/µL αCD8 | 0 | 2,5µL 6e10/µL T3 | 0 |
| CD19 PN | 20 µL 1µg/µL | 0 | 0 | 20 µL 25 ng/µL αCD19 | 2,5µL 6e10/µL T4 | 0 |

### Purification

No further purification was performed.

### Contacting tagged particles with sample

### Amount of sample

200µL, 1000 PBMCs/µL in RPMI with 10% fetal calf serum was used.

### Amount of tagged particle

2 µL of each of the tagged particles synthetized according to Table 5 was used.

### Conditions during contact

Cells were suspended in 200 µl RPMI + 10% FCS during contact. Particles were mixed with cells and incubated 30 min, r.t with gentle shaking.

### Recovering tagged particles causing specific effect/change

2 µL each of antibodies identifying cell subsets that were CD4 positive (Biolegend, Brilliant Violet 421™ anti-human CD4 Antibody, #300532), CD8 positive (Biolegend, Alexa Fluor® 700 anti-human CD8 Antibody, # 344724) and CD19 positive (BD Biosciences, APC Mouse Anti-Human CD19), were additionally added together with 0.1 µl near- IR-viability dye (Invitrogen L10119) that stains free amines. Cells were incubated 20 min, 4°C. After washing the cells twice in PBS with 2% FCS cells were fixed in 200 µL 1% paraformaldehyde O.N., 4°C.

### Wash

Before separation of cells the sample was washed in 400 µL barcode-buffer (PBS/0.5% BSA/2 mM EDTA/100 µg/ml herring DNA) followed by centrifugation 5 min, 490 g, with subsequent removal of supernatant and resuspension in 200 µL barcode-buffer.

### Separate tagged particles

Cells were sorted on a BD FACSAria equipped with three lasers (488 nm blue, 633 nm red and 405 nm violet) on the basis of the above described fluorochrome labeled anti CD4 (BV 421), anti-CD8 (Alexa Fluor 700) and anti-CD19 (APC) antibodies.

Labeled cells were sorted into tubes that had been pre-saturated overnight in 2% BSA and contained 200 µl barcode-buffer to increase the stability of the oligonucleotide tag on particles that follow with the sorted cells. The sorted cells were centrifuged 5 min, 2000 g, to allow removal of all excess buffer and to remove tagged particles not bound to cells.

### Identifying particle components by the unique particle tag

### Apply method for deconvolution of tag information

The abundance of the four oligonucleotide tags, T1 (control particle), T2 (anti-CD4 particle), T3 (anti-CD8 Particle) and T4 (anti-CD19 particle) was determined in the three individual sorted fractions by quantitative PCR using the forward prime F1 (Table 4) and the either of the four reverse primers R1 SYBR to R4 SYBR. (Table 4) together with QPCR master mix from BioRad (BioRad, SsoAdvanced™ Universal SYBR® Green Supermix, #1725270). QPCR was performed on a CFX96 Touch Real-Time PCR Detection System from BioRad using BioRad standard cycle protocol and the results were analyzed on the CFX Manager™ Software.

| Per sample mix |
|---|
| 12,5 µL 2X SYBR Green master mix |
| 0,625 µL 10 µM F primer (250nM final) |
| 0,625 µL 10 µM Rx SYBR primer (250nM final) |
| 11,25 µL Sample |
| 0 µL H2O |

Samples were set up in a 96 well plate

| | CD4 cells | CD8 cells | CD19 cells |
|---|---|---|---|
| F + R1 SYBR | sample 1 CD4 | sample 1 CD8 | sample 1 CD19 |
| F + R2 SYBR | sample 1 CD4 | sample 1 CD8 | sample 1 CD19 |
| F + R3 SYBR | sample 1 CD4 | sample 1 CD8 | sample 1 CD19 |
| F + R4 SYBR | sample 1 CD4 | sample 1 CD8 | sample 1 CD19 |

### Results

See Figure 11. Incubation of a library of DNA-tagged polystyrene (AmCyan filled) nanoparticles (PN's) with PBMCs. (a) Incubation of PN's with PBMCs. (b) FACS sorting of cells (in circle) according to presence of AmCyan (corresponding to presence of cell-associated PN's with AmCyan cargo) and respective CD4, CD8 and CD19 molecules displayed on cells. (c) QPCR analysis of DNA-tags associated with each population of sorted cells. In the CD4 cell column, the sorted CD4 cell population is analyzed for DNA-tags by QPCR. T2 has the lowest cycle of quantification (Cq) value (marked in box) indicating that T2 is predominantly associated with the isolated CD4+ cells. This is in agreement with CD4+ cells predominantly being targeted by anti-CD4 antibody-coated particles tagged with T2. In the CD8 cell column, the sorted CD8 cell population is analyzed for DNA-tags by QPCR. T3 has the lowest cycle of quantification (Cq) value (marked in box) indicating that T3 is predominantly associated with isolated CD8+ cells. This is in agreement with CD8+ cells predominantly being targeted by anti-CD8 antibody-coated PN's tagged with T3. In the CD19 cell column, the sorted CD19 cell population is analyzed for DNA-tags by QPCR. T4 has the lowest cycle of quantification (Cq) value (marked in box) indicating that T4 is predominantly associated with isolated CD19+ cells. This is in agreement with CD19+ cells predominantly being targeted by anti-CD19 antibody-coated PN's tagged with T4. T1 tagged PN's without targeting antibody was not found predominantly in any population of sorted cells.

### Conclusion on Example 8

A four member library of tagged particles can be screened on PBMC's in solution and specific cell populations can be isolated by FACS on the basis of cell surface markers and particle cargo delivered to these specific cells. Subsequently the identity of the predominant particle type associated with a given cell fraction can be revealed by analyzing the presence of associated tags in the cell fraction.

### Example 9 (E9): Synthesis and screening of a library of four tagged liposomes comprising one type of carrier, one type of cargo and one of four types of surface molecules.

### Example summary

The synthesis of tagged particles is visualized in figure 12. The results are shown in figure 13. This is an example where the sample is PBMCs.

The carrier and cargo is ready formed as a commercially available doxorubicin-loaded liposome (Dox-NP). The surface of the liposome carrier is surface modified by grafting in lipid-conjugated streptavidin. These streptavidin modified liposome carriers were further surface coated either with biotinylated anti-CD4, biotinylated anti-CD8, biotinylated anti-CD19 antibodies or no antibodies, as well as biotinylated DNA-oligonucleotide tags to respectively encode the type of surface molecule i.e the antibody specificity on the individual type of particles.

The tagged molecules were contacted with PBMCs where after CD4 positive, CD8 positive and CD19 positive cells were identified by staining with fluorochrome labeled anti CD4, anti-CD8 and anti-CD19 antibodies. CD4 positive, CD8 positive and CD19 positive populations were separated and collected by FACS. Separated fractions of cells were analyzed for associated oligonucleotide tags on cell-bound particles by SYBR green qPCR analysis using tag-specific PCR primers.

### Preparation of sample

In this example, the sample was prepared as PBMCs from peripheral blood.

### Collecting sample

Blood was obtained from the Danish blood bank (Dept. clinical immunology, 'Rigshospitalet', Denmark).

### Modifying sample

As for example 8.

### Production of tagged particles

### Synthesis

In this example, the tagged particle is a liposome (carrier) loaded with doxorubicin (cargo), which is surface modified with lipidated streptavidin and subsequently conjugated either with biotinylated anti-CD4, biotinylated anti-CD8, biotinylated anti-CD19 antibodies or no antibodies (surface molecule). Finally, the particle is further complexed with a biotinylated oligonucleotide (tag) to encode the identity of the particle.
- ***Carrier*:** Pegylated liposomes (carrier) ready loaded with doxorubicin (cargo) was purchased from Avanti Polar Lipids (Dox-NP® # 300102 concentration ∼1,5E11 liposomes/mL = 0,25 nM, 2mg/mL Dox (∼4 mM), 20 mM lipid, info from supplier). The carriers are approximately 100 nm.
- ***Cargo*:** Doxorubicin ready pre-loaded into carrier by supplier.
- ***Surface molecule*:** Biotinylated anti-CD4 antibody [RPA-T4] (#300504 from BioLegend), biotinylated anti-CD8 antibody [MEM-31] (#ab28090 from AbCam) and biotinylated anti-CD19 antibody [HIB19] (#302203 from BioLegend).

Streptavidin (# S4762 Sigma-Aldrich) was lipidated by reacting with DSPE-PEG-NHS, MW 3400, (#PG2-DSNS-3k from Nanaocs) in a molar ratio of approximately 1:2. Briefly, streptavidin was dissolved to 1 mg/mL in PBS pH 7,2. DSPE-PEG-NHS was dissolve to 1 mM in H2O. 20 µL, 1 mM DSPE-PEG-NHS was added to 1 mL, 1 mg/mL streptavidin in PBS, pH 7,2. The reaction was incubated for 1 h at 30 degrees to allow lipidation-reaction on streptavidin.
- **Tag:** T1, T2, T3 and T4 as from table 4. Synthetized by DNA technology, Aarhus, Denmark. As described in table 4, example 8.

### Synthesis of tagged particles

A library of four types of DNA-tagged liposome carriers with doxorubicin as cargo and targeting antibodies as surface molecules.

Lipidated streptavidin was grafted onto Dox-NP by mixing in a molar ratio of 1:10000 (20µL Dox-NP (20 mM) + 4 µL 10 µM lipidated streptavidin + 36 µL PBS) and incubated in a heating block (Thermomixer comfort) for 1 hour at 60 °C, where after the suspension was cooled down.

In individual Eppendorf tubes for each of the four types, liposome particles comprising streptavidin on their surface were coated either with biotinylated anti-CD4 antibody, anti-CD8 antibody, anti-CD19 antibody or no antibody respectively according to volumes and concentrations in Table 6 (and figure 12). Reagents were incubated at room temperature for 30 minutes. Then DNA-tags encoding the surface molecules were attched to particles according to volumes and concentrations in Table 6. Reagents were incubated for a further 30 minutes at room temperature. The result was four different types of DNA-tagged liposome particles.

**Table 6**

| Particle name | Strep modif Dox-NP (6,6 mM lipid, 1,3 mM Dox, 0,7 µM Strep) | Bio. anti CD4 (0,5 mg/mL) | Bio. anti CD8 (1 mg/mL) | Bio. anti CD19 (0,5 mg/mL) | Tag |
|---|---|---|---|---|---|
| Dox-NP control | 15 µL | 0 | 0 | 0 | 1µL 10µM T1 |
| Dox-NP CD4 | 15 µL | 3 µL (3,3 µM) αCD4 | 0 | | 1µL 10µM T2 |
| Dox-NP CD8 | 15 µL | 0 | 1,5 µL (6,6 µM) αCD8 | 0 | 1µL 10µM T3 |
| Dox-NP CD19 | 15 µL | 0 | 0 | 3 µL (3,3 µM) αCD19 | 1µL 10µM T4 |

### Purification

No further purification was performed.

### Contacting tagged particles with sample

### Amount of sample

200µL, 1000 PBMCs/µL in RPMI with 10% fetal calf serum was used.

### Amount of tagged particle

2 µL of each of the tagged particles synthetized according to Table 6 was used.

### Conditions during contact

Cells were suspended in 200 µl RPMI + 10% FCS during contact. Particles were mixed with cells and incubated 30 min, r.t with gentle shaking.

### Recovering tagged particles causing specific effect/change

2 µL each of antibodies identifying cell subsets that were CD4 positive (Biolegend, Brilliant Violet 421™ anti-human CD4 Antibody, #300532), CD8 positive (Biolegend, Alexa Fluor® 700 anti-human CD8 Antibody, # 344724) and CD19 positive (BD Biosciences, APC Mouse Anti-Human CD19), were additionally added. Cells were incubated for a further 20 min, 4°C. After washing the cells twice in PBS with 2% FCS cells were fixed in 200 µL 1% paraformaldehyde O.N., 4°C.

### Wash

Before separation of cells the sample was washed in 400 µL barcode-buffer (PBS/0.5% BSA/2 mM EDTA/100 µg/ml herring DNA) followed by centrifugation 5 min, 490 g, with subsequent removal of supernatant and resuspension in 200 µL barcode-buffer.

### Separate tagged particles

Cells were sorted on a BD FACSAria equipped with three lasers (488 nm blue, 633 nm red and 405 nm violet) on the basis of the above described fluorochrome labeled anti CD4 (BV 421), anti-CD8 (Alexa Fluor 700) and anti-CD19 (APC) antibodies.

Labeled cells were sorted into tubes that had been pre-saturated overnight in 2% BSA and contain 200 µl barcode-buffer to increase the stability of the oligonucleotide tag on particles that follow with the sorted cells. The sorted cells were centrifuged 5 min, 2000 g, to allow removal of all excess buffer and to remove tagged particles not bound to cells.

### Identifying particle components by the unique particle tag

### Apply method for deconvolution of tag information

The abundance of the four oligonucleotide tags, T1 (control particle), T2 (anti-CD4 particle), T3 (anti-CD8 Particle) and T4 (anti-CD19 particle) was determined in the individual sorted fractions by quantitative PCR using the forward prime F1 (Table 4) and either of the reverse primers R1 SYBR to R4 SYBR (Table 4) together with QPCR master mix from BioRad (BioRad, SsoAdvanced™ Universal SYBR® Green Supermix, #1725270). QPCR was performed on a CFX96 Touch Real-Time PCR Detection System from BioRad according to BioRad standard cycle protocol and the results were analyzed on the CFX Manager™ Software.

| Per sample mix |
|---|
| 12,5 µL 2X SYBR Green master mix |
| 0,625 µL 10 µM F primer (250nM final) |
| 0,625 µL 10 µM Rx SYBR primer (250nM final) |
| 11,25 µL Sample |
| 0 µL H2O |

Samples were set up in a 96 well plate

| | CD4 cells | CD8 cells | CD19 cells |
|---|---|---|---|
| F + R1 SYBR | sample 2 CD4 | sample 2 CD8 | sample 2 CD19 |
| F + R2 SYBR | sample 2 CD4 | sample 2 CD8 | sample 2 CD19 |
| F + R3 SYBR | sample 2 CD4 | sample 2 CD8 | sample 2 CD19 |
| F + R4 SYBR | sample 2 CD4 | sample 2 CD8 | sample 2 CD19 |

### Results

See Figure 13.

Screening of a library of four tagged liposome particles (Dox-NP's) comprising one type of carrier (liposome), one type of cargo (doxorubicin) and one of four types of surface molecules (none, anti-CD4, anti-CD8 and or anti-CD19). (a) Incubation of Dox-NP library with PBMCs. (b) FACS sorting of cells (in circle) according to respective CD4, CD8 and CD19 molecules displayed on cells. (c) QPCR analysis of DNA-tags associated with each population of sorted cells. In the CD4 cell column, the sorted CD4 cell population is analyzed for DNA-tags by QPCR. T2 has the lowest cycle of quantification (Cq) value (marked in box) indicating that T2 is predominantly associated with the isolated CD4+ cells. This is in agreement with CD4+ cells predominantly being targeted by anti-CD4 antibody-coated Dox-NP's tagged with T2. In the CD8 cell column, the sorted CD8 cell population is analyzed for DNA-tags by QPCR. T3 has the lowest cycle of quantification (Cq) value (marked in box) indicating that T3 is predominantly associated with isolated CD8+ cells. This is in agreement with CD8+ cells predominantly being targeted by anti-CD8 antibody-coated Dox-NP's tagged with T3. In the CD19 cell column, the sorted CD19 cell population is analyzed for DNA-tags by QPCR. T4 has the lowest cycle of quantification (Cq) value (marked in box) indicating that T4 is predominantly associated with isolated CD19+ cells. This is in agreement with CD19+ cells predominantly being targeted by anti-CD19 antibody-coated Dox-NP's tagged with T4. T1 tagged Dox-NP's without targeting antibody was not found predominantly in any population of sorted cells.

### Conclusion on Example 9

A four member library of tagged liposomal particles containing a relevant cancer drug (doxorubicin) can be screened on PBMC's in solution and specific cell populations can be sorted and collected by FACS on the basis of cell surface markers. Subsequently the identity of the predominant type of doxorubicin-loaded liposomal particle associated with a given cell fraction can be successfully revealed by analyzing the presence of tags of associated with the isolated cell fraction.

### Example 10 (E10): Cargo by split and mix synthesis

The precursor for library synthesis was a short, covalently linked DNA duplex-the "headpiece". The headpiece was appended with Fmoc-15-amino-4,7,10,13-tetraoxapentadecanoic acid (AOP), which served as a spacer between the DNA and the small-molecule portion of the construct. After deprotection, the amine would serve as the starting point for the synthesis of the library. The nonlinked end of the headpiece duplex contained a two-base 3' overhang, which formed a substrate for subsequent ligation to coding tags. The coding tags were short double-stranded DNA sequences consisting of a 7-base variable region flanked by constant 3' overhangs. The overhangs, which served as "sticky ends" for ligation, were unique to each cycle of synthesis, so that each set of tags could only ligate to the set from the preceding cycle, and not to truncated sequences.

We designed two related libraries based on a triazine scaffold. In both cases, 192 Fmoc amino acids were acylated onto the headpiece. After deprotection, the triazine was installed with cyanuric chloride. For DEL-A, the remaining chlorines on the triazine were substituted stepwise with 192 amines at each position, giving a three cycle library of 7,077,888 components. Among the amines at cycle 2, we included 3-amino-4-methyl-N-methoxybenzamide (AMMB), a known pharmacophore fragment for p38 mitogen-activated protein kinase (MAPK). For DEL-B, a small set of 32 amino acids was incorporated in cycle 2, and the number of amines used in cycles 3 and 4 was increased to 340 and 384, respectively, yielding a final library size of 802,160,640 over 4 cycles.

We conducted library synthesis using a split-and-pool strategy in 96-well plates. The starting duplex was arrayed into wells and ligated to cycle 1 DNA tags using T4 ligase. Following gel analysis of all wells to confirm quantitative ligation, the DNA was precipitated with ethanol. The pellets were then redissolved in buffer and subjected to acylation with Fmoc-amino acids. Progress of the chemistry was checked in a subset of wells by LCMS. After completion, the wells were pooled. In most cases, the pooled product was purified using reverse-phase HPLC. The product was again split into plates for entry into the next cycle of synthesis. After the last cycle of synthesis, a 30-base-pair primer sequence was ligated onto the library. This primer included a short randomized region that served as a control for PCR artifacts during subsequent sequencing.

## Claims

1. A method for the identification of individual components of a particle, the method comprising the steps of:
i. providing a plurality of tagged particles comprising at least one component from each of a)-c):
a) a carrier (P) selected from the group consisting of liposomes, polymeric particles, micelles, albumin complexes, dendrimers, metal colloids and ceramics,
b) a cargo molecule (K) selected from the group consisting of drugs, oligonucleotides, proteins, antibodies, radioisotopes, markers, metal ions, adjuvants, organic molecules, small molecules and cytokines, and
c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
and an oligonucleotide tag comprising one oligonucleotide tag sub-segment (p, k, t, z) for each component from a)-c),
ii. contacting said plurality of tagged particles with a sample,
iii. evaluating the ability of the tagged particles to induce biological, morphological, chemical, biochemical, catalytic, physical and/or physiological changes on the sample,
iv. identifying one or more tagged particles causing a specific change,
v. recovering from the sample said one or more tagged particles causing the specific change, and
vi. identifying by their oligonucleotide tag the at least one component from each of a)-c) of said one or more recovered tagged particles.

2. The method according to claim 1, wherein one or more components are formed from two or more component precursors.

3. The method according to any one of the preceding claims, wherein the oligonucleotide tag comprises one oligonucleotide tag sub-segment for each different component or component precursor.

4. The method according to any one of the preceding claims, wherein the oligonucleotide tag sub-segments are not physically connected to the component or component precursor they encode.

5. The method according to any one of the preceding claims, wherein the oligonucleotide tag comprises polymers selected from the group consisting of DNA, RNA, LNA and PNA or derivates or mimics thereof.

6. The method according to any one of the preceding claims, wherein the carrier is a liposome or a polymeric particle.

7. The method according to any one of the preceding claims, wherein the cargo is a selected from the group consisting of a drug, a radioisotope and a therapeutic oligonucleotide.

8. The method according to any one of the preceding claims, wherein the surface molecule is an antibody.

9. The method according to any one of the preceding claims, wherein the sample is selected from the group consisting of organisms, biological fluids, tissues, organs, cells and metastases.

10. The method according to claim 9, wherein the organism is selected from the group consisting of a mammal, a primate or a human, preferably a human.

11. A split and mix method for the production of a plurality of tagged particles, the method comprising at least the step of:
i. mixing one component from either of a)-c):
a) a carrier (P) selected from the group consisting of liposomes, polymeric particles, micelles, albumin complexes, dendrimers, metal colloids and ceramics,
b) a cargo molecule (K) selected from the group consisting of drugs, oligonucleotides, proteins, antibodies, radioisotopes, markers, metal ions, adjuvants, organic molecules, small molecules and cytokines, and
c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
with an oligonucleotide tag sub-segment (p, k, t, z) to form a first solution of first generation tagged particles,
ii. splitting said first solution of first generation tagged particles into two or more first solutions of first generation tagged particles,
iii. mixing said two or more first solutions of first generation tagged particles with one component from either of a)-c) and an oligonucleotide tag sub-segment (p, k, t, z) to form two or more second solutions of second generation tagged particles,
iv. splitting said second solution of second generation tagged particles into two or more second solutions of second generation tagged particles,
v. mixing said two or more second solutions of second generation tagged particles with one component from either of a)-c) and an oligonucleotide tag sub-segment (p, k, t, z) to form two or more third solutions of third generation tagged particles,
and wherein the plurality of tagged particles resulting from step v) comprise at least one component from each of a)-c).

12. The split and mix method according to claim 11, wherein steps iv)-v) are repeated one or more times to form further generations of tagged particles.

13. The split and mix method according to any one of claims 11-12, wherein the particle components are assembled from two or more component precursors that each are encoded by an oligonucleotide tag sub-segment.

14. A tagged particle comprising at least one component from each of a)-c):
a) a carrier (P) selected from the group consisting of liposomes, polymeric particles, micelles, albumin complexes, dendrimers, metal colloids and ceramics,
b) a cargo molecule (K) selected from the group consisting of drugs, oligonucleotides, proteins, antibodies, radioisotopes, markers, metal ions, adjuvants, organic molecules, small molecules and cytokines, and
c) a surface molecule (Z, T) selected from the group consisting of antibodies, antibody fragments, antibody mimics, peptides, proteins, ligands, aptamers, polymers, drugs, organic molecules, small molecules, sugars, oligonucleotides, carbohydrates and linkers,
and an oligonucleotide tag comprising one oligonucleotide tag sub-segment (p, k, t, z) for each component from a)-c).

15. The tagged particle according to claim 14, wherein
a) the carrier is a liposome,
b) the cargo is a drug, radioisotope or therapeutic oligonucleotide, and
c) the surface molecule is an antibody,
and the oligonucleotide tag is a DNA oligonucleotide tag comprising one DNA oligonucleotide tag sub-segment for each component from a)-c).

## Patentansprüche

1. Verfahren zur Identifizierung von einzelnen Komponenten eines Partikels, wobei das Verfahren die folgenden Schritte umfasst:
i. Bereitstellen einer Vielzahl von markierten Partikeln, umfassend zumindest eine Komponente von jedem von a)-c):
a) einen Träger (P), ausgewählt aus der Gruppe bestehend aus Liposomen, Polymerpartikeln, Mizellen, Albuminkomplexen, Dendrimeren, Metallkolloiden und Keramiken,
b) ein Frachtmolekül (K), ausgewählt aus der Gruppe bestehend aus Medikamenten, Oligonukleotiden, Proteinen, Antikörpern, Radioisotopen, Markern, Metallionen, Adjuvanzien, organischen Molekülen, kleinen Molekülen und Zytokinen, und
c) ein Oberflächenmolekül (Z, T), ausgewählt aus der Gruppe bestehend aus Antikörpern, Antikörperfragmenten, Antikörpermimika, Peptiden, Proteinen, Liganden, Aptameren, Polymeren, Medikamenten, organischen Molekülen, kleinen Molekülen, Zuckern, Oligonukleotiden, Kohlenhydraten und Linkern,
und eine Oligonukleotidmarkierung, umfassend ein Oligonukleotidmarkierungs-Untersegment (p, k, t, z) für jede Komponente aus a)-c),
ii. Inkontaktbringen der Vielzahl von markierten Partikeln mit einer Probe,
iii. Bewerten der Fähigkeit der markierten Partikel, biologische, morphologische, chemische, biochemische, katalytische, physische und/oder physiologische Änderungen der Probe zu induzieren,
iv. Identifizieren von einem oder mehreren markierten Partikeln, die eine spezifische Änderung verursachen,
v. Rückgewinnen des einen oder der mehreren markierten Partikel, die die spezifische Änderung verursachen, aus der Probe, und
vi. Identifizieren, durch deren Oligonukleotidmarkierung, der zumindest einen Komponente von jedem von a)-c) des einen oder der mehreren rückgewonnenen Partikel.

2. Verfahren nach Anspruch 1, wobei eine oder mehrere Komponenten aus zwei oder mehr Komponentenvorläufern gebildet sind.

3. Verfahren nach einem der vorhergehenden Ansprüche, wobei die Oligonukleotidmarkierung ein Oligonukleotidmarkierungs-Untersegment für jede unterschiedliche Komponente oder jeden unterschiedlichen Komponentenvorläufer umfasst.

4. Verfahren nach einem der vorhergehenden Ansprüche, wobei die Oligonukleotidmarkierungs-Untersegmente mit der Komponente oder dem Komponentenvorläufer, die/den sie kodieren, nicht physisch verbunden sind.

5. Verfahren nach einem der vorhergehenden Ansprüche, wobei die Oligonukleotidmarkierung Polymere umfasst, ausgewählt aus der Gruppe bestehend aus DNA, RNA, LNA und PNA oder Derivaten oder Mimika davon.

6. Verfahren nach einem der vorhergehenden Ansprüche, wobei der Träger ein Liposom oder ein Polymerpartikel ist.

7. Verfahren nach einem der vorhergehenden Ansprüche, wobei die Fracht ausgewählt ist aus der Gruppe bestehend aus einem Medikament, einem Radioisotop und einem therapeutischen Oligonukleotid.

8. Verfahren nach einem der vorhergehenden Ansprüche, wobei das Oberflächenmolekül ein Antikörper ist.

9. Verfahren nach einem der vorhergehenden Ansprüche, wobei die Probe ausgewählt ist aus Organismen, biologischen Fluiden, Geweben, Organen, Zellen und Metastasen.

10. Verfahren nach Anspruch 9, wobei der Organismus ausgewählt ist aus der Gruppe bestehend aus einem Säuger, einem Primaten oder einem Menschen, bevorzugt einem Menschen.

11. Ein Aufteilungs- und Mischverfahren für die Produktion einer Vielzahl von markierten Partikeln, wobei das Verfahren zumindest die folgenden Schritte umfasst:
i. Mischen einer Komponente von einem von a)-c):
a) einen Träger (P), ausgewählt aus der Gruppe bestehend aus Liposomen, Polymerpartikeln, Mizellen, Albuminkomplexen, Dendrimeren, Metallkolloiden und Keramiken,
b) ein Frachtmolekül (K), ausgewählt aus der Gruppe bestehend aus Medikamenten, Oligonukleotiden, Proteinen, Antikörpern, Radioisotopen, Markern, Metallionen, Adjuvanzien, organischen Molekülen, kleinen Molekülen und Zytokinen, und
c) ein Oberflächenmolekül (Z, T), ausgewählt aus der Gruppe bestehend aus Antikörpern, Antikörperfragmenten, Antikörpermimika, Peptiden, Proteinen, Liganden, Aptameren, Polymeren, Medikamenten, organischen Molekülen, kleinen Molekülen, Zuckern, Oligonukleotiden, Kohlenhydraten und Linkern,
mit einem Oligonukleotidmarkierungs-Untersegment (p, k, t, z), um eine erste Lösung von markierten Partikeln einer ersten Generation zu bilden,
ii. Aufteilen der ersten Lösung von markierten Partikeln der ersten Generation in zwei oder mehr erste Lösungen von markierten Partikeln der ersten Generation,
iii. Mischen der zwei oder mehr ersten Lösungen von markierten Partikeln der ersten Generation mit einer Komponente von einem von a)-c) und einem Oligonukleotidmarkierungs-Untersegment (p, k, t, z), um zwei oder mehr zweite Lösungen von markierten Partikeln einer zweiten Generation zu bilden,
iv. Aufteilen der zweiten Lösung von markierten Partikeln der zweiten Generation in zwei oder mehr zweite Lösungen von markierten Partikeln der zweiten Generation,
v. Mischen der zwei oder mehr zweiten Lösungen von markierten Partikeln der zweiten Generation mit einer Komponente von einem von a)-c) und einem Oligonukleotidmarkierungs-Untersegment (p, k, t, z), um zwei oder mehr dritte Lösungen von markierten Partikeln einer dritten Generation zu bilden,
und wobei die Vielzahl von markierten Partikeln aus Schritt v) zumindest eine Komponente von jedem von a)-c) umfasst.

12. Aufteilungs- und Mischverfahren nach Anspruch 11, wobei die Schritte iv)-v) ein Mal oder mehrere Male wiederholt werden, um weitere Generationen von markierten Partikeln zu bilden.

13. Aufteilungs- und Mischverfahren nach einem der Ansprüche 11-12, wobei die Partikelkomponenten aus zwei oder mehr Komponentenvorläufern zusammengesetzt sind, die jeweils durch ein Oligonukleotidmarkierung-Untersegment kodiert sind.

14. Markierter Partikel, umfassend zumindest eine Komponente von jedem von a)-c):
a) einen Träger (P), ausgewählt aus der Gruppe bestehend aus Liposomen, Polymerpartikeln, Mizellen, Albuminkomplexen, Dendrimeren, Metallkolloiden und Keramiken,
b) ein Frachtmolekül (K), ausgewählt aus der Gruppe bestehend aus Medikamenten, Oligonukleotiden, Proteinen, Antikörpern, Radioisotopen, Markern, Metallionen, Adjuvanzien, organischen Molekülen, kleinen Molekülen und Zytokinen, und
c) ein Oberflächenmolekül (Z, T), ausgewählt aus der Gruppe bestehend aus Antikörpern, Antikörperfragmenten, Antikörpermimika, Peptiden, Proteinen, Liganden, Aptameren, Polymeren, Medikamenten, organischen Molekülen, kleinen Molekülen, Zuckern, Oligonukleotiden, Kohlenhydraten und Linkern,
und eine Oligonukleotidmarkierung, umfassend ein Oligonukleotidmarkierungs-Untersegment (p, k, t, z) für jede Komponente aus a)-c).

15. Markierter Partikel nach Anspruch 14, wobei
a) der Träger ein Liposom ist,
b) die Fracht ein Medikament, ein Radioisotop oder ein therapeutisches Oligonukleotid ist, und
c) das Oberflächenmolekül ein Antikörper ist,
und die Oligonukleotidmarkierung eine DNA-Oligonukleotidmarkierung ist, umfassend ein DNA-Oligonukleotidmarkierungs-Untersegment für jede Komponente aus a)-c).

## Revendications

1. Procédé destiné à l'identification de composants individuels d'une particule, le procédé comprenant les étapes de :
i. fourniture d'une pluralité de particules marquées comprenant au moins un composant de chacun de a) à c) :
a) un support (P) sélectionné parmi le groupe constitué par des liposomes, des particules polymériques, des micelles, des complexes d'albumine, des dendrimères, des colloïdes métalliques et des céramiques,
b) une molécule cargo (K) sélectionnée parmi le groupe constitué par des médicaments, des oligonucléotides, des protéines, des anticorps, des isotopes radioactifs, des marqueurs, des ions métalliques, des adjuvants, des molécules organiques, des petites molécules et des cytokines, et
c) une molécule de surface (Z, T) sélectionnée parmi le groupe constitué par des anticorps, des fragments d'anticorps, des mimétiques d'anticorps, des peptides, des protéines, des ligands, des aptamères, des polymères, des médicaments, des molécules organiques, des petites molécules, des sucres, des oligonucléotides, des hydrates de carbone et des segments de liaison,
et un marqueur oligonucléotidique comprenant un sous-segment de marqueur oligonucléotidique (p, k, t, z) pour chaque composant de a) à c),
ii. la mise en contact de ladite pluralité de particules marquées avec un échantillon,
iii. l'évaluation de la capacité des particules marquées à induire des modifications biologiques, morphologiques, chimiques, biochimiques, catalytiques, physiques et/ou physiologiques sur l'échantillon,
iv. l'identification d'une ou de plusieurs particules marquées causant une modification spécifique,
v. la récupération à partir de l'échantillon desdites une ou plusieurs particules marquées provoquant la modification spécifique, et
vi. l'identification par leur marqueur oligonucléotidique de l'au moins un composant de chacun de a) à c) desdites une ou plusieurs particules marquées récupérées.

2. Procédé selon la revendication 1, dans lequel un ou plusieurs composants sont formés à partir de deux précurseurs de composants ou plus.

3. Procédé selon l'une quelconque des revendications précédentes, dans lequel le marqueur oligonucléotidique comprend un sous-segment de marqueur oligonucléotidique pour chaque composant ou précurseur de composant différent.

4. Procédé selon l'une quelconque des revendications précédentes, dans lequel les sous-segments de marqueur oligonucléotidique ne sont pas connectés physiquement au composant ou au précurseur de composant pour lequel ils codent.

5. Procédé selon l'une quelconque des revendications précédentes, dans lequel le marqueur oligonucléotidique comprend des polymères sélectionnés parmi le groupe constitué par un ADN, un ARN, un ANL et un ANP ou des dérivés ou mimétiques de ceux-ci.

6. Procédé selon l'une quelconque des revendications précédentes, dans lequel le support est un liposome ou une particule polymérique.

7. Procédé selon l'une quelconque des revendications précédentes, dans lequel le cargo est sélectionné parmi le groupe constitué par un médicament, un isotope radioactif et un oligonucléotide thérapeutique.

8. Procédé selon l'une quelconque des revendications précédentes, dans lequel la molécule de surface est un anticorps.

9. Procédé selon l'une quelconque des revendications précédentes, dans lequel l'échantillon est sélectionné parmi le groupe constitué par des organismes, des fluides biologiques, des tissus, des organes, des cellules et des métastases.

10. Procédé selon la revendication 9, dans lequel l'organisme est sélectionné parmi le groupe constitué par un mammifère, un primate ou un humain, préférablement un humain.

11. Procédé de division et de mélange destiné à la production d'une pluralité de particules marquées, le procédé comprenant au moins les étapes de :
i. mélange d'un composant de l'un ou l'autre de a) à c) :
a) un support (P) sélectionné parmi le groupe constitué par des liposomes, des particules polymériques, des micelles, des complexes d'albumine, des dendrimères, des colloïdes métalliques et des céramiques,
b) une molécule cargo (K) sélectionnée parmi le groupe constitué par des médicaments, des oligonucléotides, des protéines, des anticorps, des isotopes radioactifs, des marqueurs, des ions métalliques, des adjuvants, des molécules organiques, des petites molécules et des cytokines, et
c) une molécule de surface (Z, T) sélectionnée parmi le groupe constitué par des anticorps, des fragments d'anticorps, des mimétiques d'anticorps, des peptides, des protéines, des ligands, des aptamères, des polymères, des médicaments, des molécules organiques, des petites molécules, des sucres, des oligonucléotides, des hydrates de carbone et des segments de liaison,
avec un sous-segment de marqueur oligonucléotidique (p, k, t, z) pour former une première solution d'une première génération de particules marquées,
ii. division de ladite première solution de particules marquées de la première génération en deux premières solutions de particules marquées de la première génération ou plus,
iii. mélange desdites deux premières solutions de particules marquées de la première génération ou plus avec un composant de l'un ou l'autre de a) à c) et un sous-segment de marqueur oligonucléotidique (p, k, t, z) pour former deux deuxièmes solutions de particules marquées de deuxième génération ou plus,
iv. division de ladite deuxième solution de particules marquées de la deuxième génération en deux deuxièmes solutions de particules marquées de la deuxième génération ou plus,
v. mélange desdites deux deuxièmes solutions de particules marquées de la deuxième génération ou plus avec un composant de l'un ou l'autre de a) à c) et un sous-segment de marqueur oligonucléotidique (p, k, t, z) pour former deux troisièmes solutions de particules marquées de troisième génération ou plus,
et dans lequel la pluralité de particules marquées résultant de l'étape v) comprend au moins un composant de chacun de a) à c).

12. Procédé de division et de mélange selon la revendication 11, dans lequel les étapes iv) à v) sont répétées une ou plusieurs fois pour former des générations supplémentaires de particules marquées.

13. Procédé de division et de mélange selon l'une quelconque des revendications 11 à 12, dans lequel les composants de particules sont assemblés à partir de deux précurseurs de composants ou plus qui sont chacun codés par un sous-segment de marqueur oligonucléotidique.

14. Particule marquée comprenant au moins un composant parmi chacun de a) à c) :
a) un support (P) sélectionné parmi le groupe constitué par des liposomes, des particules polymériques, des micelles, des complexes d'albumine, des dendrimères, des colloïdes métalliques et des céramiques,
b) une molécule cargo (K) sélectionnée parmi le groupe constitué par des médicaments, des oligonucléotides, des protéines, des anticorps, des isotopes radioactifs, des marqueurs, des ions métalliques, des adjuvants, des molécules organiques, des petites molécules et des cytokines, et
c) une molécule de surface (Z, T) sélectionnée parmi le groupe constitué par des anticorps, des fragments d'anticorps, des mimétiques d'anticorps, des peptides, des protéines, des ligands, des aptamères, des polymères, des médicaments, des molécules organiques, des petites molécules, des sucres, des oligonucléotides, des hydrates de carbone et des segments de liaison,
et un marqueur oligonucléotidique comprenant un sous-segment de marqueur oligonucléotidique (p, k, t, z) pour chaque composant de a) à c).

15. Particule marquée selon la revendication 14, dans laquelle
a) le support est un liposome,
b) la molécule cargo est un médicament, un isotope radioactif ou un oligonucléotide thérapeutique, et
c) la molécule de surface est un anticorps,
et le marqueur oligonucléotidique est un marqueur oligonucléotidique d'ADN comprenant un sous-segment de marqueur oligonucléotidique d'ADN pour chaque composant de a) à c).


### SEQUENCE LISTING

<110> Danmark Tekniske Univeristet
   Reker Hadrup, Sine
   Lyngsø, Christina
   Nyboe Jakobsen, Søren
<120> High throughput optimization of content-loaded nanoparticles
<130> P55443PC01
<160> 84
<170> PatentIn version 3.5
<210> 1
   <211> 25
   <212> DNA
   <213> Artificial
<220>
   <223> DNA sequence
<400> 1
   tagctctgta cgtctatgcg aaagt 25
<210> 2
   <211> 22
   <212> DNA
   <213> Artificial
<220>
   <223> DNA sequence
<400> 2
   gttgtaccta agtagagact gc 22
<210> 3
   <211> 75
   <212> DNA
   <213> Artificial
<220>
   <223> DNA sequence
<400> 3
   gggctcccgg attttgtaag aatgaattga ggtggcgtag tcccatctga ttagttgtac 60
   ctaagtagag actgc 75
<210> 4
   <211> 75
   <212> DNA
   <213> Artificial
<220>
   <223> DNA sequence
<400> 4
   gggctcccgg attttgtaag aatgatatcg attcaccaac gcagacgcat ttagttgtac 60
   ctaagtagag actgc 75
<210> 5
   <211> 75
   <212> DNA
   <213> Artificial
<220>
   <223> DNA sequence
<400> 5
   gggctcccgg attttgtaag aatgaaaact ggtatgcgag acgcaggatg ttagttgtac 60
   ctaagtagag actgc 75
<210> 6
   <211> 63
   <212> DNA
   <213> Artificial
<220>
   <223> DNA sequence
<400> 6
   ccatctcatc cctgcgtgtc tccgactcag tggaagatcg ggctcccgga ttttgtaaga 60
   atg 63
<210> 7
   <211> 63
   <212> DNA
   <213> Artificial
<220>
   <223> DNA sequence
<400> 7
   ccatctcatc cctgcgtgtc tccgactcag tgtcctgata ggctcccgga ttttgtaaga 60
   atg 63
<210> 8
   <211> 45
   <212> DNA
   <213> Artificial
<220>
   <223> DNA sequence
<400> 8
   cctctctatg ggcagtcggt gatgttgtac ctaagtagag actgc 45
<210> 9
   <211> 24
   <212> DNA
   <213> Artificial
<220>
   <223> DNA sequence
<400> 9
   cagatgggac tacgccacct caat 24
<210> 10
   <211> 25
   <212> DNA
   <213> Artificial
<220>
   <223> DNA sequence
<400> 10
   atgcgtctgc gttggtgaat cgata 25
<210> 11
   <211> 25
   <212> DNA
   <213> Artificial
<220>
   <223> DNA sequence
<400> 11
   catcctgcgt ctcgcatacc agttt 25
<210> 12
   <211> 25
   <212> DNA
   <213> Artificial
<220>
   <223> DNA sequence
<400> 12
   atcgagacat gcagatacgc tttca 25
<210> 13
   <211> 45
   <212> DNA
   <213> Artificial
<220>
   <223> DNA sequence
<400> 13
   taactccacc gcatcagggt agactgacac tatttctcct actag 45
<210> 14
   <211> 49
   <212> DNA
   <213> Artificial
<220>
   <223> DNA sequence
<400> 14
   agctctgtac gtctatgcga aagtattgag gtggcgtagt cccatctga 49
<210> 15
   <211> 45
   <212> DNA
   <213> Artificial
<220>
   <223> DNA sequence
<400> 15
   taactccacc gcatcagggt agactggatt ctccggccct actag 45
<210> 16
   <211> 49
   <212> DNA
   <213> Artificial
<220>
   <223> DNA sequence
<400> 16
   agctctgtac gtctatgcga aagtattgag gtggcgtagt cccatctga 49
<210> 17
   <211> 45
   <212> DNA
   <213> Artificial
<220>
   <223> DNA sequence
<400> 17
   taactccacc gcatcagggt agacttatag caggtatcct actag 45
<210> 18
   <211> 49
   <212> DNA
   <213> Artificial
<220>
   <223> DNA sequence
<400> 18
   agctctgtac gtctatgcga aagtattgag gtggcgtagt cccatctga 49
<210> 19
   <211> 45
   <212> DNA
   <213> Artificial
<220>
   <223> DNA sequence
<400> 19
   taactccacc gcatcagggt agactagctc atcgcaacct actag 45
<210> 20
   <211> 49
   <212> DNA
   <213> Artificial
<220>
   <223> DNA sequence
<400> 20
   agctctgtac gtctatgcga aagtattgag gtggcgtagt cccatctga 49
<210> 21
   <211> 45
   <212> DNA
   <213> Artificial
<220>
   <223> DNA sequence
<400> 21
   taactccacc gcatcagggt agactgattc atcatatcct actag 45
<210> 22
   <211> 49
   <212> DNA
   <213> Artificial
<220>
   <223> DNA sequence
<400> 22
   agctctgtac gtctatgcga aagtattgag gtggcgtagt cccatctga 49
<210> 23
   <211> 45
   <212> DNA
   <213> Artificial
<220>
   <223> DNA sequence
<400> 23
   taactccacc gcatcagggt agactaagta ttacgatcct actag 45
<210> 24
   <211> 49
   <212> DNA
   <213> Artificial
<220>
   <223> DNA sequence
<400> 24
   agctctgtac gtctatgcga aagtattgag gtggcgtagt cccatctga 49
<210> 25
   <211> 45
   <212> DNA
   <213> Artificial
<220>
   <223> DNA sequence
<400> 25
   taactccacc gcatcagggt agactacggg tagttatcct actag 45
<210> 26
   <211> 49
   <212> DNA
   <213> Artificial
<220>
   <223> DNA sequence
<400> 26
   agctctgtac gtctatgcga aagtattgag gtggcgtagt cccatctga 49
<210> 27
   <211> 45
   <212> DNA
   <213> Artificial
<220>
   <223> DNA sequence
<400> 27
   taactccacc gcatcagggt agactatatg ggttatacct actag 45
<210> 28
   <211> 49
   <212> DNA
   <213> Artificial
<220>
   <223> DNA sequence
<400> 28
   agctctgtac gtctatgcga aagtattgag gtggcgtagt cccatctga 49
<210> 29
   <211> 45
   <212> DNA
   <213> Artificial
<220>
   <223> DNA sequence
<400> 29
   taactccacc gcatcagggt agactggcgt tagttttcct actag 45
<210> 30
   <211> 49
   <212> DNA
   <213> Artificial
<220>
   <223> DNA sequence
<400> 30
   agctctgtac gtctatgcga aagtattgag gtggcgtagt cccatctga 49
<210> 31
   <211> 45
   <212> DNA
   <213> Artificial
<220>
   <223> DNA sequence
<400> 31
   taactccacc gcatcagggt agactagaaa tccggaacct actag 45
<210> 32
   <211> 49
   <212> DNA
   <213> Artificial
<220>
   <223> DNA sequence
<400> 32
   agctctgtac gtctatgcga aagtattgag gtggcgtagt cccatctga 49
<210> 33
   <211> 20
   <212> DNA
   <213> Artificial
<220>
   <223> DNA sequence
<400> 33
   cttcgtgata tgatacagct 20
<210> 34
   <211> 20
   <212> DNA
   <213> Artificial
<220>
   <223> DNA sequence
<400> 34
   ggatgatcga agcactatac 20
<210> 35
   <211> 20
   <212> DNA
   <213> Artificial
<220>
   <223> DNA sequence
<400> 35
   gacaaagccc gtatacagct 20
<210> 36
   <211> 20
   <212> DNA
   <213> Artificial
<220>
   <223> DNA sequence
<400> 36
   ggatgatcct gtttcgggca 20
<210> 37
   <211> 20
   <212> DNA
   <213> Artificial
<220>
   <223> DNA sequence
<400> 37
   ggctatgatt taatacagct 20
<210> 38
   <211> 20
   <212> DNA
   <213> Artificial
<220>
   <223> DNA sequence
<400> 38
   ggatgatccc gatactaaat 20
<210> 39
   <211> 20
   <212> DNA
   <213> Artificial
<220>
   <223> DNA sequence
<400> 39
   cataagctcc tgatacagct 20
<210> 40
   <211> 20
   <212> DNA
   <213> Artificial
<220>
   <223> DNA sequence
<400> 40
   ggatgatcgt attcgaggac 20
<210> 41
   <211> 20
   <212> DNA
   <213> Artificial
<220>
   <223> DNA sequence
<400> 41
   ctcataaacc agatacagct 20
<210> 42
   <211> 20
   <212> DNA
   <213> Artificial
<220>
   <223> DNA sequence
<400> 42
   ggatgatcga gtatttggtc 20
<210> 43
   <211> 20
   <212> DNA
   <213> Artificial
<220>
   <223> DNA sequence
<400> 43
   ctcgccttcg gaatacagct 20
<210> 44
   <211> 20
   <212> DNA
   <213> Artificial
<220>
   <223> DNA sequence
<400> 44
   ggatgatcga gcggaagcct 20
<210> 45
   <211> 20
   <212> DNA
   <213> Artificial
<220>
   <223> DNA sequence
<400> 45
   cttaaaactt gtatacagct 20
<210> 46
   <211> 20
   <212> DNA
   <213> Artificial
<220>
   <223> DNA sequence
<400> 46
   ggatgatcga attttgaaca 20
<210> 47
   <211> 20
   <212> DNA
   <213> Artificial
<220>
   <223> DNA sequence
<400> 47
   gctcataatt acatacagct 20
<210> 48
   <211> 20
   <212> DNA
   <213> Artificial
<220>
   <223> DNA sequence
<400> 48
   ggatgatccg agtattaatg 20
<210> 49
   <211> 20
   <212> DNA
   <213> Artificial
<220>
   <223> DNA sequence
<400> 49
   ccggctaggt atatacagct 20
<210> 50
   <211> 20
   <212> DNA
   <213> Artificial
<220>
   <223> DNA sequence
<400> 50
   ggatgatcgg ccgatccata 20
<210> 51
   <211> 20
   <212> DNA
   <213> Artificial
<220>
   <223> DNA sequence
<400> 51
   ggatacatct caatacagct 20
<210> 52
   <211> 20
   <212> DNA
   <213> Artificial
<220>
   <223> DNA sequence
<400> 52
   ggatgatccc tatgtagagt 20
<210> 53
   <211> 20
   <212> DNA
   <213> Artificial
<220>
   <223> DNA sequence
<400> 53
   gaagatcctt actatcgcac 20
<210> 54
   <211> 20
   <212> DNA
   <213> Artificial
<220>
   <223> DNA sequence
<400> 54
   tatgtcgact tctaggaatg 20
<210> 55
   <211> 20
   <212> DNA
   <213> Artificial
<220>
   <223> DNA sequence
<400> 55
   aggaactgcg cctatcgcac 20
<210> 56
   <211> 20
   <212> DNA
   <213> Artificial
<220>
   <223> DNA sequence
<400> 56
   tatgtcgatc cttgacgcgg 20
<210> 57
   <211> 20
   <212> DNA
   <213> Artificial
<220>
   <223> DNA sequence
<400> 57
   tgcgggttac cgtatcgcac 20
<210> 58
   <211> 20
   <212> DNA
   <213> Artificial
<220>
   <223> DNA sequence
<400> 58
   tatgtcgaac gcccaatggc 20
<210> 59
   <211> 20
   <212> DNA
   <213> Artificial
<220>
   <223> DNA sequence
<400> 59
   tggaaccaag actatcgcac 20
<210> 60
   <211> 20
   <212> DNA
   <213> Artificial
<220>
   <223> DNA sequence
<400> 60
   tatgtcgaac cttggttctg 20
<210> 61
   <211> 20
   <212> DNA
   <213> Artificial
<220>
   <223> DNA sequence
<400> 61
   agtcctgaga cttatcgcac 20
<210> 62
   <211> 20
   <212> DNA
   <213> Artificial
<220>
   <223> DNA sequence
<400> 62
   tatgtcgatc aggactctga 20
<210> 63
   <211> 20
   <212> DNA
   <213> Artificial
<220>
   <223> DNA sequence
<400> 63
   tccatttatg cgtatcgcac 20
<210> 64
   <211> 20
   <212> DNA
   <213> Artificial
<220>
   <223> DNA sequence
<400> 64
   tatgtcgaag gtaaatacgc 20
<210> 65
   <211> 20
   <212> DNA
   <213> Artificial
<220>
   <223> DNA sequence
<400> 65
   tactaaatca cttatcgcac 20
<210> 66
   <211> 20
   <212> DNA
   <213> Artificial
<220>
   <223> DNA sequence
<400> 66
   tatgtcgaat gatttagtga 20
<210> 67
   <211> 20
   <212> DNA
   <213> Artificial
<220>
   <223> DNA sequence
<400> 67
   gaagtcatta tatatcgcac 20
<210> 68
   <211> 20
   <212> DNA
   <213> Artificial
<220>
   <223> DNA sequence
<400> 68
   tatgtcgact tcagtaatat 20
<210> 69
   <211> 20
   <212> DNA
   <213> Artificial
<220>
   <223> DNA sequence
<400> 69
   tcctactacc tgtatcgcac 20
<210> 70
   <211> 20
   <212> DNA
   <213> Artificial
<220>
   <223> DNA sequence
<400> 70
   tatgtcgaag gatgatggac 20
<210> 71
   <211> 20
   <212> DNA
   <213> Artificial
<220>
   <223> DNA sequence
<400> 71
   ttgcctcatc agtatcgcac 20
<210> 72
   <211> 20
   <212> DNA
   <213> Artificial
<220>
   <223> DNA sequence
<400> 72
   tatgtcgaaa cggagtagtc 20
<210> 73
   <211> 25
   <212> DNA
   <213> Artificial
<220>
   <223> DNA sequence
<400> 73
   gttcagatga cgccgcgcta tctgt 25
<210> 74
   <211> 16
   <212> DNA
   <213> Artificial
<220>
   <223> DNA sequence
<400> 74
   atagcgtgca agtcta 16
<210> 75
   <211> 22
   <212> DNA
   <213> Artificial Sequence
<220>
   <223> Artificial
<400> 75
   agctctgtac gtctatgcga aa 22
<210> 76
   <211> 75
   <212> DNA
   <213> Artificial Sequence
<220>
   <223> Artificial
<220>
   <221> Biotin-tag
   <222> (1)..(1)
   <223> biotin-C6 on 5' terminal nucleic acid
<400> 76
   tagctctgta cgtctatgcg aaagtattga ggtggcgtag tcccatctga gacgtgcatg 60
   ctaacagtcc ttata 75
<210> 77
   <211> 75
   <212> DNA
   <213> Artificial Sequence
<220>
   <223> Artificial
<220>
   <221> Biotin-tag
   <222> (1)..(1)
   <223> biotin-C6 on 5' terminal nucleic acid
<400> 77
   tagctctgta cgtctatgcg aaagttatcg attcaccaac gcagacgcat gacgtgcatg 60
   ctaacagtcc ttata 75
<210> 78
   <211> 75
   <212> DNA
   <213> Artificial Sequence
<220>
   <223> Artificial
<220>
   <221> Biotin-tag
   <222> (1)..(1)
   <223> biotin-C6 on 5' terminal nucleic acid
<400> 78
   tagctctgta cgtctatgcg aaagtaaact ggtatgcgag acgcaggatg gacgtgcatg 60
   ctaacagtcc ttata 75
<210> 79
   <211> 75
   <212> DNA
   <213> Artificial Sequence
<220>
   <223> Artificial
<220>
   <221> Biotin-tag
   <222> (1)..(1)
   <223> biotin-C6 on 5' terminal nucleic acid
<400> 79
   tagctctgta cgtctatgcg aaagtccatc tgcatatggc ccatgactca gacgtgcatg 60
   ctaacagtcc ttata 75
<210> 80
   <211> 21
   <212> DNA
   <213> Artificial Sequence
<220>
   <223> Artificial
<400> 80
   cacgtctcag atgggactac g 21
<210> 81
   <211> 16
   <212> DNA
   <213> Artificial Sequence
<220>
   <223> Artificial
<400> 81
   acgtcatgcg tctgcg 16
<210> 82
   <211> 17
   <212> DNA
   <213> Artificial Sequence
<220>
   <223> Artificial
<400> 82
   cacgtccatc ctgcgtc 17
<210> 83
   <211> 18
   <212> DNA
   <213> Artificial Sequence
<220>
   <223> Artificial
<400> 83
   cacgtctgag tcatgggc 18
<210> 84
   <211> 25
   <212> DNA
   <213> Artificial
<220>
   <223> Artificial sequence
<220>
   <221> Lipidated anchor
   <222> (25)..(25)
   <223> DSPE-PEG attached to 3' terminal nucleic acid
<400> 84
   tagctctgta cgtctatgcg aaagt 25